# EUROPÄISCHE PATENTSCHRIFT

(11) **EP 2 376 650 B1**
(45) Veröffentlichungstag und Bekanntmachung des Hinweises auf die Patenterteilung: **02.01.2013**
(21) Anmeldenummer: 09795414.3
(22) Anmeldetag: 17.12.2009
(51) Int. Cl.: C12Q 1/68, G01N 33/58, G01N 33/542

(54) **NACHWEISKONJUGAT UND VERFAHREN ZU POLYCHROMATISCHEN ANALYSE**
DETECTION CONJUGATE AND METHOD FOR POLYCHROMATIC ANALYSIS
CONJUGUÉ DE DÉTECTION ET PROCÉDÉ D'ANALYSE POLYCHROMATIQUE

(30) Priorität: 17.12.2008 DE 102008062372
(43) Veröffentlichungstag der Anmeldung: 19.10.2011
(73) Patentinhaber: Medizinische Hochschule Hannover, 30625 Hannover (DE)
(72) Erfinder: HENNIG, Christian, 30625 Hannover (DE); HANSEN, Gesine, 30625 Hannover (DE)
(74) Vertreter: Taruttis, Stefan Georg
(86) Internationale Anmeldenummer: PCT/EP2009/067384
(87) Internationale Veröffentlichungsnummer: WO 2010/070037

(56) Entgegenhaltungen:
- WO-A1-2004/057023
- WO-A1-2007/101706
- WO-A2-2006/002167
- US-A1- 2004 219 526
- HENNIG CHRISTIAN ET AL: "A Versatile Platform for Comprehensive Chip-Based Explorative Cytometry" CYTOMETRY, Bd. 75A, Nr. 4, 11. November 2008 (2008-11-11), Seiten 362-370, XP008121600
- MITTAG ANJA ET AL: "Hyperchromatic cytometry principles for cytomics using slide based cytometry" CYTOMETRY, Bd. 69A, Nr. 7, Juli 2006 (2006-07), Seiten 691-703, XP002578625
- TÁRNOK ATTILA: "Slide-based cytometry for cytomics--a minireview" CYTOMETRY. PART A, JOHN WILEY, HOBOKEN, NJ, US LNKD- DOI:10.1002/CYTO.A.20317, Bd. 69, Nr. 7, 1. Juli 2006 (2006-07-01), Seiten 555-562, XP002562649 ISSN: 1552-4922 [gefunden am 2006-06-19]
- MITTAG ANJA ET AL: "Sequential photobleaching of fluorochromes for polychromatic slide-based cytometry." CYTOMETRY. PART A : THE JOURNAL OF THE INTERNATIONAL SOCIETY FOR ANALYTICAL CYTOLOGY MAR 2006 LNKD- PUBMED:16479599, Bd. 69, Nr. 3, März 2006 (2006-03), Seiten 139-141, XP002578626 ISSN: 1552-4922

### SEQUENCE LISTING

<110> Medizinische Hochschule Hannover Hennig, Dr., Christian Hansen, Prof. Dr., Gesine
<120> Nachweiskonjugat und Verfahren zur Analyse
<130> M1009PCT
<160> 4
<170> PatentIn version 3.5
<210> 1
   <211> 20
   <212> DNA
   <213> Artificial
<220>
   <223> /note="oligonukleotide linker, Seq.-ID No. 1"
<400> 1
   tttttttttt gttttttttt 20
<210> 2
   <211> 21
   <212> DNA
   <213> Artificial
<220>
   <223> /note="hybridising oligonukleotide, Seq.-ID No. 2"
<400> 2
   aaaaaaaaaa caaaaaaaaa a 21
<210> 3
   <211> 25
   <212> DNA
   <213> Artificial Sequence
<220>
   <223> /note="synthetic aptamer, Seq.-ID No. 3"
<400> 3
   gcagttgatc ctttggatac cctgg 25
<210> 4
   <211> 33
   <212> DNA
   <213> Artificial Sequence
<220>
   <223> /note="synthetic aptamer, Seq.-ID No. 4"
<400> 4
   ucguaugggu gggaucggga agggcuacga aca 33

## Beschreibung

Die Erfindung betrifft Nachweiskonjugate mit einem Antikörperanteil, Verfahren zur Detektion von Analyten mittels eines Nachweiskonjugats und die Verwendung der Nachweiskonjugate in Verfahren zum spezifischen Nachweis von Analyten, z.B. von spezifischen Antigenen, die in Lösung, extrazellulär oder intrazellulär an suspendierten oder an einem Trägersubstrat gebundenen Zellen oder an einem Trägersubstrat gebunden sind, und insbesondere an lebenden Zellen oder an denaturierten und permeabilisierten Zellen, die in Suspension oder an ein Trägersubstrat gebunden sind, angeordnet sind. Nachweiskonjugate enthalten als Alternative zum Antikörperanteil einen für einen Analyten spezifischen Bindeanteil, z.B. einen Rezeptorligand, einen Rezeptor, ein spezifisch bindendes Molekül, z.B. ein Lektin, Biotin, Avidin, ein Oligosaccharid, jeweils als Bindeanteil des Nachweiskonjugats.

Erfindungsgemäße Nachweiskonjugate umfassen einen Bindeanteil und einen mit diesem verbundenen Fluorochromanteil, der mit einem ersten Ende eines Linkers verbunden ist. An dem dem ersten Ende gegenüberliegenden zweiten Ende des Linkers kann der Bindeanteil angeordnet sein, so dass der Linker zwischen Bindeanteil und Fluorochromanteil angeordnet ist. Die Nachweiskonjugate zeichnen sich dadurch aus, dass der am Fluorochromanteil des Konjugats angebundene Linker eingerichtet ist, die optische Aktivität des Fluorochromanteils des Konjugats durch Wechselwirkung mit einem Reagenz unwirksam zu machen. In einer ersten Ausführungsform kann der Bindeanteil mit dem Fluorochromanteil verbunden sein, während der Linker mit seinem ersten Ende mit dem Fluorochromanteil verbunden ist.

Die erfindungsgemäße Einrichtung des Linkers dazu, bei Anwesenheit eines Reagenzes, das eine mit dem Linker spezifisch reaktive Verbindung ist, die optische Aktivität des Fluorochromanteils unwirksam zu machen, erlaubt Nachweisverfahren, bei denen gleichzeitig und/oder nacheinander zumindest zwei Nachweiskonjugate mit verschiedener Spezifität verwendet werden, jeweils mit dem Schritt des für einen Linker spezifischen Unwirksammachens der optischen Aktivität des Fluorochromanteils im Konjugat, z.B. nach Detektion eines Nachweiskonjugats, sodass z.B. bei Verwendung in Alternative zum Bleichen bei gleichen und verschiedenen Fluorochromanteilen der Konjugate keine wechselseitige Störung auftritt, beispielsweise Interferenz oder die wechselseitige Anregung von Fluorochromen unterbleibt. Auf diese Weise wird eine unspezifische Minderung des optischen Signals eines Fluorochroms oder die Erzeugung eines unspezifischen optischen Signals von einem Konjugat vermieden, das von einem vorherigen Analyseschritt zurückbleibt. Vorzugsweise haben die Linker von Konjugaten, die nacheinander oder gleichzeitig mit derselben Probe kontaktiert werden, unterschiedliche Bindespezifitäten für ein jeweils passend spezifisches Reagenz zum Unwirksammachen des Fluorochromanteils.

### Stand der Technik

Mahnke und Roederer (Clin. Lab. Med. 2007, 27(3) (2007)) beschreiben, wie eine Mehrzahl von Antikörperkonjugaten für den Nachweis von Antigenen auf Zellen in der Durchflusszytometrie aufeinander abgestimmt werden können, um beim gleichzeitigen Einsatz Interferenzen der Fluorochromanteile zu vermeiden, die jeweils unterschiedliche Emissionsspektren haben, um unterscheidbar zu sein. Zur Trennung überlappender Emissionsspektren, insbesondere bei unspezifischer Anregung eines Fluorochroms durch Energietransfer von einem anderen Fluorochrom, wird eine rechnergestützte Auswertung der Signale vorgeschlagen, bei der unspezifische Emissionssignale als Hintergrund abgezogen werden können.

Hennig, Adams und Hansen, Cytrometry Part A, 362 - 370 (2009) beschreiben die Analyse trägergebundener Zellen mit nacheinander folgender Markierung mittels fluoreszenzmarkierter Antikörper. Zwischen einzelnen Makierungsschritten mit einem Antikörper wurden die Fluoreszenzfarbstoffe durch Bleichen inaktiviert.

Mittag et al., Cytometry Part A, 691 - 703 (2006) beschreiben die Analyse von Zellen in der Durchflusszyometrie mit gleichzeitiger Färbung der Zellen mit bis zu acht Fluorochromen, die an verschiedene Antikörper konjugiert sind. Die Unterscheidung der Fluorochrome erfolgt durch nacheinander folgende Messungen mit jeweils unterschiedlichen Wellenlängen und Filtern zur Detektion, wobei die Daten anschließend übereinander gelegt werden.

Die WO 2004/057023 A1 beschreibt die Analyse von Nukleinsäuren durch Bindung an immobilisierte Fänger - Oligonukleotide und Bindung an farbstoffmarkierte Oligonukleotide, wobei ungebundene farbstoffmarkierte Oligonukleotide durch spezifische komplementäre Oligonukleotide mit einem Quencher optisch inaktiviert werden.

Die WO 2006/002167 A2 beschreibt eine Nachweissonde mit einer Haamadelstruktur, an deren hybridisierenden Enden einerseits Fluoreszenzmoleküle und andererseits Quencher gebunden sind. Die Interaktion des nicht hybridisierten Anteils der Haarnadelstruktur an einen Analyten führt zur Veränderung der optischen Aktivität der Fluoreszenzmarkierung.

Die US 2004/0219526 A1 beschreibt einen Sandwich - ELISA, bei dem der Fängerantikörper mittels eines abschnittsweise doppelsträngigen Oligonukleotids an den Träger gebunden ist.

Laffers et al. (Cytometry Part A 69A: 127-130 (2006)) beschreiben die Analyse von Zellen per DURCHFLUSSZYTOMETRIE oder immobilisierter Zellen mit Antikörper-Fluorochrom-Konjugaten, die jeweils unterschiedliche Fluorochrome aufwiesen. Für die Auswertung wurden die Daten aus der Durchflusszytometrie (DZ) oder Mikroskopie mit Fluoreszenzdetektion computergestützt überlagert.

Tárnok (Cytometry Part A 69A: 555-562 (2006)) erwähnt neben der Zytometrie unter Verwendung von Antikörperkonjugaten mit verschiedenen Fluorochromen die nacheinander folgende Anwendung von Antikörper-Fluorochrom-Konjugaten, das Ausbleichen von Fluorochromen mittels Bestrahlung, die Aktivierung und die Zerstörung von Fluorochromen mittels Strahlung.

Perfetto et al. (Nature 648-654 (2004)) beschreiben Durchflusszytometer für die Detektion einer Vielzahl von Fluorochromen durch jeweils sehr kurz nacheinander erfolgende spezifische Anregung und Wellenlängen-spezifische Detektion emittierten Lichts, was als gleichzeitige Messung bezeichnet wird. Es wird gezeigt, dass verschiedene Fluorochrome, die gleichzeitig in einer Probe vorliegen, bei Anregung mit einer Wellenlänge, die für das Fluorochrom spezifisch ist, dennoch unspezifische Emissionen zeigen, was auf einen Energietransfer unter den Fluorochromen zurückgeführt wird. Zur Vermeidung unspezifischer Signale wird die Abstimmung der gleichzeitig einzusetzenden Antikörper-Fluorochrom-Konjugate in einem empirischen Verfahren vorgeschlagen. Zur weiteren Verminderung unspezifischer Emissionssignale wird eine mathematische Kompensation vorgeschlagen, mit der Emissionen herausgerechnet werden, die auf den unspezifischen Energietransfer zwischen Fluorochromen zurückgehen können.

Die US 6,150,173 beschreibt einen rechnergesteuerten Pipettierautomaten, mit dem jeweils nacheinander Antikörper-Fluorochrom-Konjugate mit einer Probe inkubiert werden, nach Einstrahlung einer Anregungswellenlänge die emittierte Strahlung gemessen wird, anschließend das Fluorochrom durch Bestrahlung mit UV zerstört und ein neues Antikörper-Fluorochrom-Konjugat zu der selben Probe gegeben wird.

Die WO 2007/101706 A1 beschreibt, dass vor der Detektion eines spezifischen Antikörper-Fluorochrom-Konjugats die biologische Probe durch Bestrahlung behandelt wird, um unspezifische Fluoreszenz zu verringern.

An den vorgenannten Verfahren zum Nachweis mehrerer Antikörper-Fluorochrom-Konjugate ist nachteilig, dass Fluorochrome unspezifisch Licht emittieren können, beispielsweise nach Energietransfer von einem anderen Fluorochrom, wenn mehrere unterschiedliche Fluorochrome gleichzeitig zur Analyse einer Probe verwendet werden. Bei Verfahren, in denen Antikörper-Fluorochrom-Konjugate nacheinander mit einer Probe in Kontakt gebracht werden, wobei jeweils ein Fluorochrom durch Bestrahlung zerstört wird, ist nachteilig, dass einerseits zum Zerstören von Fluorochromen ein hoher Energieeintrag die Probe verändern kann, andererseits die Zerstörung eines Fluorochroms nicht vollständig bzw. nicht irreversibel erfolgt, sodass häufig auch im Anschluss an eine solche zerstörende Bestrahlung unspezifische Emissionen von Fluorochromen festgestellt werden kann, was auch als Regeneration der Fluorochrome bezeichnet wird.

Weiterhin ist an Verfahren, Fluorochrome durch Bestrahlung unwirksam zu machen die verhältnismäßig lange Dauer der Bestrahlung nachteilig.

### Aufgabe der Erfindung

Gegenüber dem bekannten Stand der Technik ist es Aufgabe der Erfindung, Reagenzien mit einem Konjugat mit einem spezifisch einen Analyten bindenden Bindeanteil und einem Fluorochromanteil (auch als Fluorophor oder Fluoreszenzfarbstoffbezeichnet) bereitzustellen, mit dem die Nachteile des Standes der Technik vermieden werden, insbesondere ein Bindeanteil-Fluorochrom-Konjugat, insbesondere ein Antikörper-Fluorochrom-Konjugat bereitzustellen, das eingerichtet ist, dass bei gleichzeitiger und/oder sequenzieller Kontaktierung einer Probe mit zumindest zwei Bindeanteil-Fluorochromanteil-Konjugaten die Wirkung eines Fluorochroms gezielt inaktivierbar ist.

Weiterhin ist es Aufgabe der Erfindung, ein Verfahren zur Analyse unter Verwendung der Reagenzien bereitzustellen, bei dem zwischen der Kontaktierung der Probe mit verschiedenen Bindeanteil-Fluorochrom- Konjugaten die optische Aktivität zumindest eines Fluorochroms unwirksam gemacht wird, insbesondere mit Konjugaten, deren Bindeanteile verschieden und deren Fluorochromanteile identisch sind. Es wird angestrebt, dass das Analyseverfahren die Bestrahlung zum Unwirksammachen von Fluorochromen durch eine gezielte Wechselwirkung eines Reagenzes mit dem Fluorochrom ersetzt.

### Allgemeine Beschreibung der Erfindung

Die Erfindung löst die vorgenannten Aufgaben mit den Merkmalen der Ansprüche und stellt insbesondere eine Zusammenstellung von Reagenzien bereit, die auch als Testkit bezeichnet wird, das ein Nachweiskonjugat mit einem Bindeanteil und ein Reagenz zum Unwirksammachen des Fluorochromanteils durch Wechselwirkung mit dem Linker enthält. Der Bindeanteil ist insbesondere ein Antikörperanteil. Das Nachweiskonjugat weist einen mit dem Antikörperanteil verbundenen Fluorochromanteil und einen mit dem Fluorochromanteil verbundenen Linker auf, wobei der Linker ein Oligonukleotid umfasst oder daraus besteht. Vorzugsweise ist der Linker mit seinem ersten Ende mit dem Fluorochromanteil verbunden. Optional ist der Linker mit seinem zweiten, dem ersten Ende gegenüberliegenden Ende mit dem Bindeanteil verbunden, so dass der Linker Bindeanteil und Fluorochrom miteinander verbindet. Da der Linker ein Oligonukleotid aufweist oder daraus besteht, wird der Linker auch als Oligonukleotidlinker bezeichnet. Das Nachweiskonjugat kann aus dem Bindeanteil, der vorzugsweise ein natürlicher oder synthetischer Antikörper ist, dem Fluorochrom und dem Oligonukleotidlinker bestehen. Für die Zwecke der Erfindung umfasst der Begriff Oligonukleotid generell ein- und doppelsträngige DNA und/oder RNA und synthetische Oligonukleotidsequenzen, beispielsweise PNA (Peptidnukleinsäure), insbesondere mit einer Länge im Bereich von 6 bis 200 Nukleotiden, bevorzugt 20 bis 100 Nukleotiden.

Für die nachfolgende Beschreibung wird der Begriff Antikörper stellvertretend für Bindeanteil verwendet, und der BegriffNachweiskonjugat oder Antikörperkonjugat wird auch stellvertretend für Bindeanteil-Konjugate verwendet, die anstelle des Antikörperanteils einen anderen Bindeanteil aufweisen, der z.B. aus der Gruppe ausgewählt ist, die aus Oligonukleinsäuren, Rezeptorliganden, Rezeptoren, Lektinen, Oligosacchariden, koordinativen Komplexen, Antigenen, z.B. Aptameren, Peptiden, die für vorbestimmte MHCI oder MHCII spezifisch sind, MHC-Tetramere, Biotin, Avidin und Paratop bildenden Antikörperfragmenten besteht. Ein Bindeanteil weist eine Affinität zu einem Analyten auf, der ein Bindungspartner des Bindeanteils ist. Als Bindeanteil hoher Affinität sind Antikörper und deren Paratop bildende Abschnitte bevorzugt, sowie Aptamere. Ein Aptamer, das Bindeanteil ist, besteht vorzugsweise aus einzelsträngiger DNA oder RNA, z.B. mit oder aus der Sequenz 5'-GCAGTTGATCCTTTGGATACCCTGG-3' (Seq.-ID Nr. 3), die für den Tumormarker MUC1 S1.3 spezifisch ist, oder 5'-UCGUAUGGGUGGGAUCGGGAAGGGCUACGAACA-3' (Seq.-ID Nr. 4), die für den Maus-CD30 (Lymphom-Marker) spezifisch ist.

In einer ersten Ausfiihrungsform enthält die Zusammenstellung von Reagenzien neben dem ersten Reagenz, das das Nachweiskonjugat enthält, als zweites Reagenz, das den Fluorochromanteil des Nachweiskonjugats bei Kontaktieren unwirksam macht, einen Quencher, der mit einem Oligonukleotid verbunden ist, das spezifisch mit dem Oligonukleotid des Oligonukleotidlinkers hybridisierbar ist, und das insbesondere einen Abschnitt aufweist, der eine zum Oligonukleotidlinker revers komplementäre Basenfolge hat. Vorzugsweise hat das Oligonukleotid des Quenchers eine zur Hybridisierung an den Oligonukleotidlinker ausreichende Länge, z.B. von 10 bis 30 nt Länge. Der Abschnitt des Oligonukleotidlinkers, an den das mit dem Quencher verbundene Oligonukleotid hybridisierbar ist, wird auch als Quencherabschnitt bezeichnet.

Vorzugsweise verbindet der Oligonukleotidlinker den Bindeanteil mit dem Fluorochromanteil, jedoch kann der Oligonukleotidlinker alternativ nur an den Fluorochromanteil gebunden sein, und der Fluorochromanteil kann direkt oder mittels einer Bindungsgruppe mit dem Bindeanteil verbunden sein. Denn die erfindungsgemäße Wirkung des Oligonukleotidlinkers, eine Nukleotidsequenz als Quencherabschnitt am Nachweiskonjugat bereitzustellen, an den ein hybridisierbares Oligonukleotid mit gebundenem Quencher bindet, so dass der Quencher in die Nähe des Fluorochromanteils angeordnet wird, tritt auch bei dieser Anordnung auf.

In der ersten Ausführungsform ermöglicht das als Bestandteil des Oligonukleotidlinkers in dem erfindungsgemäßen Antikörperkonjugat enthaltene Oligonukleotid das spezifische Unwirksammachen des Fluorochroms, da das am Quencher gebundene Oligonukleotid den Quencher durch Hybridisieren mit dem Quencherabschnitt des Oligonukleotidlinkers am Fluorochromanteil anordnet, und der Quencher in der Folge vom Fluorochromanteil emittierte Strahlung aufnimmt.

In der ersten Ausführungsform ist das erfindungsgemäße Antikörperkonjugat für ein Testkit und ein Nachweisverfahren mit zumindest zwei nacheinander eingesetzten Nachweiskonjugaten geeignet, weil der Fluorochromanteil dadurch unwirksam gemacht werden kann, dass dem Fluorochromanteil ein Quencher in unmittelbarer räumlicher Nähe zuordnbar ist, der in der Lage ist, vom Fluorochromanteil emittierte Strahlung aufzunehmen und strahlungslos auszulöschen. In jeder Ausführungsform und Variante der Erfindung können optional anstelle eines Antikörperkonjugats zumindest zwei, vorzugsweise 2 bis 10 oder bis 4 Antikörperkonjugate gleichzeitig eingesetzt werden. Die Einrichtung des Antikörperkonjugats dazu, dass ein Quencher dem Fluorochromanteil eines Konjugats zuordnbar ist, ist dadurch realisiert, dass der Quencher mit einem Oligonukleotid versehen ist, das mit einem Quencherabschnitt des Oligonukleotidlinkers hybridisierbar ist, und insbesondere eine zu einem Quencherabschnitt des Oligonukleotidlinkers revers komplementäre Basenfolge aufweist. Beispielsweise kann das mit dem Quencher verbundene Oligonukleotid ein mit einem Quencherabschnitt des Oligonukleotidlinkers hybridisierbares antisense-Oligonukleotid sein, z.B. RNA, DNA oder PNA, vorzugsweise ein Oligonukleotid, das in wässriger Lösung mit einem Abschnitt des Oligonukleotidlinkers hybridisiert, der dem Fluorochromanteil benachbart ist. Es hat sich gezeigt, dass die Hybridisierung eines zu einem Quencherabschnitt des Oligonukleotidlinkers revers komplementären Oligonukleotids, das mit einem Quencher verbunden ist, diesen Quencher in enge räumliche Nähe zum Fluorochromanteil des Konjugats bringt, sodass Fluorochromanteil und Quencher innerhalb des Försterradius liegen und der Energietransfer vom Fluorochromanteil auf den Quencher erfolgt, und von diesem die strahlungslose oder optisch nicht detektierbare Dissipation der Energie.

In einer zweiten Ausführungsform ist der Oligonukleotidlinker zwischen Bindeanteil und Fluorochromanteil angeordnet und das Unwirksammachen des Fluorochroms wird bei der Verwendung in Analyseverfahren durch Hydrolyse des Linkers erreicht, sodass unabhängig von der Bindung des Antikörpers an ein Antigen der Fluorochromanteil des Konjugats abgetrennt wird und dadurch für die ortsspezifische Detektion, z.B. die Emission von Strahlung am Bindungsort des Bindeanteils, unwirksam gemacht ist. Zur Hydrolyse des erfindungsgemäßen Oligonukleotidlinkers können unspezifische Hydrolasen, beispielsweise Ribonukleasen (RNasen), Desoxyribonukleasen (DNasen), insbesondere Endo-RNasen und Endo-DNasen eingesetzt werden, sowie dann, wenn der Oligonukleotidlinker eine für eine Restriktase spezifische Nukleotidsequenz enthält, die spezifische Restriktase.

Der Oligonukleotidlinker erfindungsgemäßer Bindeanteil-Konjugate kann generell kovalent oder über Kopplungsgruppen an den Bindeanteil gebunden sein, z.B. über eine Thio-, Ester-, Amid- oder Aminbindung, bzw. über zumindest bifunktionale Verbindungen kovalent oder über einen Komplex zweier spezifisch aneinander bindender, insbesondere komplexbildender Kopplungsgruppen, von denen jeweils eine mit dem Bindeanteil und eine mit dem Oligonukleotidlinker verbunden oder kovalent gebunden ist. So können Biotin und Avidin bzw. Biotin und Streptavidin Kopplungsgruppen sein, von denen jeweils eine Gruppe mit einem Ende des Oligonukleotidlinkers und die andere mit dem Bindeanteil und/oder mit dem Fluorochromanteil verbunden ist.

Eine der Bindungsmöglichkeiten des Oligonukleotidlinkers an den Bindeanteil kann in jeder Ausführungsform zur Bindung des Fluorochromanteils an den Oligonukleotidlinker verwendet sein, und unabhängig davon, in der ersten Ausführungsform zur Bindung des Quenchers an sein Oligonukleotid.

Das erfindungsgemäße Unwirksammachen des Fluorochromanteils eines Antikörper-Fluorochrom-Konjugats ermöglicht jeweils aufeinander folgende Nachweisreaktionen verschiedener Epitope oder Antigene durch den für das jeweilige Epitop spezifischen Antikörperanteil des Konjugats, wobei durch Unwirksammachen des Fluorochromanteils des Konjugats, entweder gemäß der zweiten Ausführungsform durch Abtrennen des Fluorochromanteils oder gemäß der ersten Ausführungsform durch Unterdrücken bzw. Auslöschen der Aktivität des Fluorochromanteils mittels eines Quenchers, die ortsspezifische Bindung des Bindeanteils nicht mehr durch Nachweis des Fluorochromanteils detektierbar ist. Daher kann im Nachweisverfahren anschließend ein weiteres Antikörperkonjugat mit der selben Probe kontaktiert werden, um ein weiteres spezifisches Antigen nachzuweisen, wobei der Fluorochromanteil des zuvor eingesetzten Antikörperkonjugats unwirksam ist und daher keine störenden Emissionen erzeugen kann, beispielsweise keine unspezifische Emission aufgrund von Energietransfer, während der Fluorochromanteil des weiteren Antikörperkonjugats nachweisbar ist. Auch in dieser Ausführungsform können anstelle eines Antikörperkonjugats zumindest zwei Antikörperkonjugate mit verschiedener Analytenspezifität des Bindeanteils eingesetzt werden.

Die Erfindung stellt daher ein Nachweiskonjugat und ein Verfahren zu dessen *in-situ* Herstellung, z.B. in Kontakt mit der Probe, und die Verwendung des Nachweiskonjugats zur

Analyse bereit. Das Nachweiskonjugat weist einen Bindeanteil und einen Fluorochromanteil auf, sowie einen Oligonukleotidlinker, der vorzugsweise Bindeanteil und Fluorochromanteil miteinander verbindet und abschnittsweise einzel- oder doppelsträngig sein kann. Der Oligonukleotidlinker ist in der zweiten Ausführungsform durch eine Nuklease hydrolysierbar und enthält in der bevorzugten ersten Ausführungsform einen Quencherabschnitt. Der in einer Variante der Erfindung im Oligonukleotidlinker enthaltene Abschnitt, mit dem das Oligonukleotid des hybridisierbaren Fluorochroms hybridisierbar ist, insbesondere revers komplementär ist, wird auch als Fluorochromabschnitt bezeichnet. In der doppelsträngigen Ausführung weist der Oligonukleotidlinker in seiner Nukleotidsequenz einen Fluorochromabschnitt auf, und das Fluorochrom ist mit einem Oligonukleotid verbunden, das einen zum Fluorochromabschnitt hybridisierbaren, z.B. revers komplementären Abschnitt enthält. In der bevorzugten Ausführung weist der Oligonukleotidlinker einen Quencherabschnitt auf und das Nachweiskonjugat liegt in der Zusammenstellung in Kombination mit einem hybridisierbaren Quencher vor, der durch Kontaktieren mit dem Nachweiskonjugat dessen Fluorochromanteil unwirksam macht.

Der Quencherabschnitt kann bei einem Oligonukleotidlinker, der durch Hybridisierung des hybridisierbaren Fluorochroms mit dem Fluorochromabschnitt abschnittsweise doppelsträngig ist, in dem Anteil des Oligonukleotidlinkers enthalten sein, der als zweiter Oligonukleotidlinkerabschnitt mit dem Fluorochrom verbunden ist, z.B. direkt bzw. ohne Nukleinsäurehybridisierung, oder in dem Abschnitt, der mittels Nukleinsäurehybridisierung mit dem Bindeanteil verbunden ist. So enthält z.B. der am Fluorochrom gebundene zweite Oligonukleotidlinkerabschnitt, der mit dem Fluorochromabschnitt hybridisierbar ist, den Quencherabschnitt. Ein Oligonukleotidabschnitt, der ohne Nukleinsäurehybridisierung verbunden ist, ist z.B. kovalent oder durch Komplexbildung ohne Beteiligung von Nukleinsäuren verbunden.

Bevorzugt betrifft die Erfindung ein Antikörperkonjugat, an dessen Quencherabschnitt des Oligonukleotidlinkers das Oligonukleotid eines hybridisierbaren Quenchers hybridisiert ist, und ein Verfahren zu dessen Herstellung durch Hybridisierung des Oligonukleotids eines hybridisierbaren Quenchers mit dem Quencherabschnitt des Oligonukleotidlinkers, z.B. im Verfahren zur Analyse. Im erfindungsgemäßen Verfahren kann das Antikörperkonjugat durch Kontaktieren der Probe mit den Bestandteilen des Nachweiskonjugats hergestellt werden, das bevorzugt einen Oligonukleotidlinker mit einem Quencherabschnitt aufweist, und vorzugsweise durch anschließendes Kontaktieren mit einem hybridisierbaren Quencher. Das Kontaktieren des Antikörperkonjugats mit dem hybridisierbaren Quencher führt zum Unwirksammachen des Fluorochromanteils und erlaubt die anschließende Analyse mit einem weiteren Nachweiskonjugat, das den gleichen oder einen anderen Fluorochromanteil aufweist, ohne dass Wechselwirkungen mit einem der vorhergehend oder gleichzeitig verwendeten Nachweiskonjugate auftreten, insbesondere bei Nachweiskonjugaten, deren Quencherabschnitte jeweils einzigartig bzw. die einem Bindeanteil spezifisch zugeordnet sind.

Die Ausführungsformen der Erfindung haben den Vorteil, dass mehrfach hintereinander Antikörperkonjugate mit jeweils dem selben Fluorochromanteil zur Analyse unterschiedlicher Antigene, d.h. mit unterschiedlichen Antikörperanteilen eingesetzt werden können, da jeweils vor dem Kontaktieren der Probe mit einem weiteren Antikörperkonjugat bereits in der Probe vorhandene Fluorochromanteile unwirksam gemacht sind. Auch der Oligonukleotidlinker kann bei Nachweiskonjugaten der Erfindung jeweils dieselbe Oligonukleotidsequenz aufweisen, denn eine Wechselwirkung mit Oligonukleotidlinkern oder Oligonukleotiden hybridisierbarer Quencher von Antikörperkonjugaten, die in vorherigen Schritten des Nachweisverfahrens mit der Probe kontaktiert wurden, tritt nicht im signifikanten Ausmaß auf, insbesondere nicht wegen der Waschschritte, die nach Kontaktieren mit einem Antikörperkonjugat und/oder nach Zugabe eines hybridisierbaren Quenchers bzw. einer Nuklease als Verfahrensschritt vorzugsweise durchgeführt werden.

Entsprechend sieht das erfindungsgemäße Verfahren in der ersten Ausführungsform vor, eine Probe mit dem erfindungsgemäßen Nachweiskonjugat zu kontaktieren, vorzugsweise Waschen zur Entfernung ungebundenen Nachweiskonjugats, Einstrahlen von Energie bei einer Anregungswellenlänge, um den Fluorochromanteil zur Emission von Strahlung anzuregen, Detektieren emittierter Strahlung, und anschließende Unwirksammachen des Fluorochromanteils mittels Kontaktieren mit einem hybridisierbaren Quencher. Entsprechend weist in der ersten Ausführungsform der Oligonukleotidlinker einen Quencherabschnitt auf.

In der zweiten Ausführungsform erfolgt das Unwirksammachen des Fluorochromanteils durch Hydrolysieren des Oligonukleotidlinkers, beispielsweise durch Kontaktieren mit einer Endo-Nuklease. Nach Unwirksammachen des Fluorochromanteils wird die Probe gewaschen, um ungebundenen hybridisierbaren Quencher bzw. Nuklease zu entfernen. Anschließend wird die Probe mit einem weiteren erfindungsgemäßen Nachweiskonjugat kontaktiert und dessen Fluorochromanteil detektiert, ebenfalls durch Einstrahlen einer Anregungswellenlänge und Detektion emittierter Strahlung. Es hat sich gezeigt, dass das Nachweisverfahren an einer Probe menschlicher Zellen, die auf einem Objektträger immobilisiert waren, mit mindestens 15 aufeinander folgenden Kontaktierungen mit Nachweiskonjugaten ohne nennenswerte Wechselwirkung der Nachweiskonjugate untereinander durchführbar ist.

Oligonukleotidlinker und an Quenchern gebundene Oligonukleotide, insbesondere Quencherabschnitte und Fluorochromabschnitte, haben vorzugsweise jeweils unterschiedliche einzigartige Nukleinsäuresequenzen, d.h. solche, die zueinander keine Sequenzähnlichkeit zur Hybridisierung gegenüber anderen Nukleinsäuren aufweisen und die nicht mit der zu analysierenden Probe hybridisieren, insbesondere eine Nukleinsäuresequenz, die zu Nukleinsäuren zu analysierender Zellen nicht hybridisierbar bzw. nicht komplementär ist. In Ausführungsformen, in denen der Bindeanteil ein Oligonukleotid ist, enthält der Bindeanteil zur Probe komplementäre Sequenzen, während der Oligonukleotidlinker eine nicht zur Probe komplementäre und nicht mit dieser hybridisierbare Sequenzen aufweist. Das Oligonukleotid des Quenchers hat vorzugsweise eine nicht zur Probe komplementäre und nicht mit dieser hybridisierbare Sequenz, sondern eine nur mit dem Quencherabschnitt des Oligonukleotidlinkers hybridisierbare Sequenz. Der Quencher ist so mit seinem Oligonukleotid verbunden, dass er bei Hybridisierung mit dem Quencherabschnitt des Oligonukleotidlinkers eine Anordnung in unmittelbarer Nähe des Fluorochromanteils einnimmt.

In einer Variante der Erfindung, die in der ersten und zweiten Ausführungsform möglich ist, ist das Oligonukleotid des Linkers, der den Bindeanteil mit dem Fluorochromanteil verbindet, in einem Abschnitt zweisträngig, in welchem ein mit dem Fluorochromanteil verbundener Oligonukleotidlinkerabschnitt mit einem Abschnitt des Oligonukleotidlinkers hybridisiert, der mit dem Bindeanteil verbunden ist. Der mit dem Fluorochromanteil verbundene zweite Oligonukleotidlinkerabschnitt hat eine mit dem Fluorochromabschnitt des Oligonukleotidlinkers hybridisierbare, z.B. eine revers komplementäre Nukleotidsequenz. Durch Hybridisierung des mit dem Fluorochromanteil verbundenen zweiten Oligonukleotidlinkerabschnitts mit dem ersten Oligonukleotidlinkerabschnitt, der mit dem Bindeanteil verbunden ist, weist das Konjugat einen den Fluorochromanteil mit dem Bindeanteil verbindenden Oligonukleotidlinker auf. Ein solches Bindeanteil-Oligonukleotidlinker-Fluorochromanteil-Konjugat weist durch Hybridisierung des mit dem Fluorochromanteil verbundenen zweiten Oligonukleotidlinkerabschnitts einen abschnittweise doppelsträngigen Oligonukleotidlinker auf.

Entsprechend der ersten Ausführungsform ist der Oligonukleotidlinker abschnittweise mit dem Oligonukleotid des Quenchers hybridisierbar, z.B. in einem Quencherabschnitt des ersten Oligonukleotidlinkerabschnitts, an den das mit dem Quencher verbundene Oligonukleotid hybridisierbar ist. Entsprechend der zweiten Ausführungsform ist auch der doppelsträngige Abschnitt des Oligonukleotidlinkers hydrolysierbar, so dass kein zusätzlicher einzelsträngiger Abschnitt im Oligonukleotidlinker für das Unwirksammachen erforderlich ist. Für die zweite Ausführungsform kann in dem doppelsträngigen Abschnitt des Oligonukleotidlinkers eine Erkennungssequenz einer Restriktase enthalten sein. Ein mit einem zum ersten Oligonukleotidlinkerabschnitt komplementären zweiten Oligonukleotidlinkerabschnitt versehenes Fluorochrom, auch als hybridisierbares Fluorochrom bezeichnet, verbindet sich spezifisch durch die spezifische Hybridisierung mittels seines komplementären zweiten Oligonukleotidlinkerabschnitts mit dem Fluorochromabschnitt des ersten Oligonukleotidlinkerabschnitts, der den Bindeanteil trägt und bildet dadurch das Konjugat, das einen Bindeanteil, einen Oligonukleotidlinker mit einem Abschnitt aus doppelsträngiger Nukleotidsequenz und ein Fluorochrom aufweist oder aus diesen Elementen besteht. Entsprechend besteht das Antikörper- Fluorochrom-Konjugat in dieser Variante aus zumindest einem Bindeanteil, zumindest einem Fluorochromanteil und zumindest einem diese verbindenden, abschnittsweise doppelsträngigen Oligonukleotidlinker und ist daher zum spezifischen Unwirksammachen des Fluorochromanteils geeignet, insbesondere durch Bindung eines hybridisierbaren Quenchers mit z.B. revers komplementärer Nukleotidsequenz an den Quencherabschnitt des Oligonukleotidlinkers, oder durch Hydrolyse des Oligonukleotidlinkers mittels Nuklease.

Auch in der Variante der Bindekonjugate mit einem Oligonukleotidlinker, der durch Hybridisierung eines am Fluorochromanteil gebundenen zweiten Oligonukleotidlinkerabschnitts mit dem Fluorochromabschnitt abschnittsweise doppelsträngig ist, kann das Bindekonjugat bei Durchführung des Verfahrens in Mischung mit der Probe gebildet werden. Entsprechend umfasst das Testkit der Erfindung dann ein erstes Reagenz mit einem ersten Anteil, der ein Bindeanteil-ersten Oligonukleotidlinkerabschnitt-Konjugat aufweist und, getrennt von dem ersten Anteil oder in Mischung mit dem ersten Anteil, einen zweiten Anteil, der ein Fluorochrom mit gebundenem zweitem Oligonukleotidlinkerabschnitt aufweist, der mit dem Fluorochromabschnitt des ersten Oligonukleotidlinkerabschnitts hybridisierbar ist.

In dieser Variante umfasst das Analyseverfahren vorzugsweise die Schritte des Kontaktierens einer Probe mit dem Bindeanteil-Oligonukleotidlinkerabschnitt-Konjugat, Waschen der Probe zum Entfernen ungebundenen Bindeanteil-Oligonukleotidlinkerabschnitt-Konjugats, Kontaktieren der Probe mit Fluorochromanteil, das einen mit dem Oligonukleotidlinkerabschnitt hybridisierbaren Oligonukleotidabschnitt (auch als hybridisierbares Fluorochrom bezeichnet) aufweist, und Waschen zum Entfernen des ungebundenen hybridisierbaren Fluorochroms, und die optische Detektion des Fluorochroms. Anschließend wird der Fluorochromanteil durch Kontaktieren der Probe mit einem, ein mit dem Oligonukleotidlinker hybridisierbares Oligonukleotid aufweisenden Quencher und/oder Kontaktieren mit einer Nuklease mit anschließendem Waschen unwirksam gemacht.

Der Fluorochromabschnitt und der damit hybridisierbare zweite Oligonukleotidlinkerabschnitt des hybridisierbaren Fluorochroms sowie vorzugsweise auch der Quencherabschnitt und der damit hybridisierbare Abschnitt des Oligonukleotids des hybridisierbaren Quenchers sind einzigartig, insbesondere der Fluorochromabschnitt und der Quencherabschnitt, sind vorzugsweise jeweils einzigartig und bilden ausschließlich jeweils mit den mit ihnen hybridisierbaren Abschnitten der Oligonukleotide von hybridisierbarem Fluorochrom bzw. hybridisierbaren Quencher unter den Detektionsbedingungen einen doppelsträngigen Abschnitt. Besonders bevorzugt sind der Fluorochromabschnitt und der Quencherabschnitt jeweils unter wenig stringenten Hybridisierungsbedingungen, z.B. unter physiologischen Bedingungen, einzigartig und hybridisieren nicht miteinander, insbesondere sind sie ausschließlich mit dem zweiten Oligonukleotidlinkerabschnitt des hybridisierbaren Fluorochroms bzw. dem Oligonukleotid des hybridisierbaren Quenchers hybridisierbar, und sind besonders bevorzugt nicht mit einer Nukleotidsequenz der zu analysierenden Probe hybridisierbar. Auf diese Weise erfolgt beim erfindungsgemäßen Verfahren die Ausbildung eines Oligonukleotidlinkers in dem für den Bindeanteil spezifischen Fluorochromabschnitt durch Hybridisierung mit dem zweiten Oligonukleotidlinkerabschnitt des hybridisierbaren Fluorochroms.

In dieser Ausführungsform ist der Quencherabschnitt auch dann Bestandteil des Oligonukleotidlinkers, wenn er Bestandteil des zweiten Oligonukleotidlinkerabschnitts des hybridisierbaren Fluorochroms ist. Denn bei Ausbildung des Oligonukleotidlinkers durch Hybridisierung seines Fluorochromabschnitts mit einem für den Bindeanteil spezifischen, revers komplementären Abschnitt des Oligonukleotids des hybridisierbaren Fluorochroms ist der Quencherabschnitt Bestandteil des Oligonukleotidlinkers. Die Anordnung des Quencherabschnitts auf dem zweiten Oligonukleotidlinkerabschnitt des hybridisierbaren Fluorochroms ist hier bevorzugt, da dann im erfindungsgemäßen Verfahren bei Kontaktierung einer Probe mit einer Mehrzahl von Bindeanteilen mit Oligonukleotidlinker, der einen für den Bindeanteil spezifischen einzigartigen Fluorochromabschnitt aufweist, mit nacheinander folgender Kontaktierung der Probe mit jeweils nur einem hybridisierbaren Fluorochrom, der Quencherabschnitt des hybridisierbaren Fluorochroms jeweils gleich sein kann, und entsprechend der hybridisierbare Abschnitt des Oligonukleotids des hybridisierbaren Quenchers jeweils dieselbe, zum Quencherabschnitt revers komplementäre einzigartige Nukleotidsequenz aufweisen kann.

Es hat sich gezeigt, dass der Oligonukleotidlinker jeder Ausführungsform erfindungsgemäßer Konjugate, insbesondere erfindungsgemäßer Antikörperkonjugate, sowohl bei Verfahren zur Analyse von lebenden Zellen, insbesondere tierischen Zellen und Geweben, die sowohl in Suspension als auch auf einem Substrat immobilisiert sein können, auf Grund des spezifischen Unwirksammachens des Fluorochromanteils zur mehrfach aufeinanderfolgenden Kontaktierung der Probe mit Nachweiskonjugaten geeignet ist.

Generell ist das Fluorochrom an einem ersten Ende des Quencherabschnitts des Oligonukleotidlinkers angeordnet, und entsprechend ist der Quencher mit dem Ende seines Oligonukleotids verbunden, das bei der Hybridisierung angrenzend an das erste Ende des Quencherabschnitts positioniert ist. An dem dem ersten Ende des Quencherabschnitts gegenüberliegenden zweiten Ende des Oligonukleotidlinkers ist der Bindeanteil angeordnet, oder das zweite Ende des Oligonukleotidlinkers ist ungebunden, wenn der Bindeanteil ohne zwischenliegenden Oligonukleotidlinker mit dem Fluorochrom verbunden ist.

Wenn z.B. das erste Ende des Oligonukleotidlinkers das 3'-Ende des Quencherabschnitts ist, an dem der Fluorochromanteil angeordnet ist, ist der Quencher vorzugsweise am 5'-Ende seines Oligonukleotids angebunden, bzw. an dessen 3'-Ende, wenn das erste Ende des Quencherabschnitts das 5'-Ende des Oligonukleotidlinkers ist. Vorzugsweise ist das Oligonukleotid des Quenchers zum Quencherabschnitt revers komplementär und der Quencher ist an dem Ende angeordnet, das dem Ende der Nukleotidsequenz des Quencherabschnitts gegenüberliegt, so dass bei einem Oligonukleotid des Quenchers, das revers komplementär zum Quencherabschnitt ist, der Quencher in die Nähe des Fluorochromanteils anordnbar ist.

Grundlage für das erfindungsgemäße Analyseverfahren ist jeweils der Oligonukleotidlinker, der in der zweiten Ausführungsform durch Hydrolyse des Linkers und in der bevorzugten ersten Ausführungsform mittels der spezifischen Hybridisierung eines einen Quencher tragenden Oligonukleotids mit einem Quencherabschnitt die für den Oligonukleotidlinker spezifische Inaktivierung eines Fluorochroms ermöglicht.

Insbesondere finden die erfindungsgemäßen Antikörperkonjugate bei Verfahren zur Analyse biologischer Proben Verwendung, die in Suspension oder auf einem Träger immobilisiert sind und denaturierte Antigene aufweisen, insbesondere denaturierte und/oder perforierte prokaryotische und eukaryotische Zellen und Gewebe, insbesondere tierische Zellen und Gewebe.

So finden die erfindungsgemäßen Konjugate Verwendung bei der Analyse suspendierter Analyten, einschließlich zellgebundener Antigene, beispielsweise in der Durchflusszytometrie zur Markierung lebender tierischer Zellen, optional mit Sortierung markierter Zellen in Abhängigkeit von der Detektion des Fluorochroms.

In einer bevorzugten Ausführungsform umfasst das erfindungsgemäße Analyseverfahren die zeitlich aufeinander folgende Kontaktierung einer auf einem Trägersubstrat immobilisierten biologischen Probe, insbesondere lebender tierischer Zellen, die beispielsweise durch Bindung auf einem beschichteten Trägersubstrat immobilisiert sind, mit einem erfindungsgemäßen Nachweiskonjugat. Die Immobilisierung auf dem Trägersubstrat, der vorzugsweise ein mikroskopischer Objektträger aus Glas ist, kann beispielsweise durch eine Beschichtung des Trägersubstrats vermittelt sein, die die Adsorption von Zellen fördert, z.B. eine Beschichtung mit Polylysin. Für die aufeinander folgenden Schritte der Kontaktierung der biologischen Probe mit verschiedenen wässrigen Zusammensetzungen wird das Trägersubstrat vorzugsweise von einem beabstandeten Deckel überdeckt, z.B. einem parallel zum Trägersubstrat angeordneten Deckglas. Für die Beabstandung des Deckels vom Trägersubstrat kann das Trägersubstrat und/oder der Deckel als Abstandshalter dienende Vorsprünge aufweisen, beispielsweise zwei beabstandete, etwa parallel zueinander angeordnete Vorsprünge, die zwischen sich, dem Trägersubstrat und dem Deckel einen Durchtrittskanal bilden. Die biologische Probe ist in dem Durchtrittskanal, nämlich auf dem Trägersubstrat immobilisiert bzw. fixiert und kann nacheinander von Zusammensetzungen kontaktiert werden, wobei der Nachweis von Antikörperkonjugat, das mit einem Antigen der Probe in Wechselwirkung tritt, durch Detektion der vom Fluorochromanteil emittierten Strahlung erfolgt, die in Reaktion auf eingestrahlte Strahlung mit Anregungswellenlänge entsteht. Vorzugsweise wird ungebundenes Nachweiskonjugat im Anschluss an die Kontaktierung mit der Probe durch Austausch des Mediums entfernt, das die Probe umgibt. Das Entfernen ungebundenen Antikörperkonjugats erfolgt vorzugsweise durch Durchtretenlassen eines Spülmediums durch den Durchtrittskanal, sodass nur an der Probe gebundenes Nachweiskonjugat an der immobilisierten Probe verbleibt und dort Strahlung emittiert, während ungebundenes Nachweiskonjugat durch das Waschen von der Probe entfernt ist.

Zur Immobilisierung von biologischen Proben, beispielsweise von Gewebsschnitten, kann ein Trägersubstrat mit einem Rahmen versehen sein, der als formschlüssiger Vorsprung auf dem Trägersubstrat angeordnet ist und beispielsweise eine Höhe von 0,8- bis 5-mal der Schichtdicke der Gewebsprobe hat, vorzugsweise eine Höhe von 1- bis 1,5-mal der Schichtdicke der Gewebsprobe, während besonders bevorzugt der Deckel in einem Abstand zur Gewebsprobe und dem Rahmen angeordnet ist. Auf diese Weise ermöglicht der formschlüssig um eine Gewebsprobe angeordnete Rahmen auf dem Trägersubstrat den Durchtritt wässriger Zusammensetzungen durch den Durchtrittskanal, der zwischen Trägersubstrat und Deckel gebildet ist, sodass wässrige Zusammensetzungen, beispielsweise Waschlösungen und Zusammensetzungen, die das erfindungsgemäße Nachweiskonjugat enthalten, zu der fixierten Gewebsprobe und von dieser weg strömen gelassen werden können.

Weiterhin können biologische Proben, insbesondere tierische Zellen und Gewebsproben in herkömmlicher Weise auf einem Trägersubstrat denaturiert und/oder perforiert fixiert sein.

Das erfindungsgemäße Verfahren zur Analyse erlaubt durch die Immobilisierung bzw. Fixierung der biologischen Probe die Überlagerung zeitlich nacheinander detektierter Signale verschiedener Konjugate, insbesondere mikroskopischer Abbildungen, die von den an der Probe angeordneten Konjugaten emittierte Strahlung enthalten. Besonders vorteilhaft erlauben es die Immobilisierung und die Fixierung der biologischen Probe in einem Durchtrittskanal, dass die Probe während aufeinander folgender Detektionsschritte, beispielsweise mit Antikörperkonjugaten mit jeweils unterschiedlichem Antikörperanteil, die Probe in einer festgelegten Position im Aufnahmebereich eines Detektors, beispielsweise im Gesichtsfeld eines Mikroskops zu belassen, oder die Probe durch identische Anordnung des Trägersubstrats im Erfassungsbereich des Detektors, beispielsweise dem Gesichtsfeld eines Mikroskops jeweils in der identischen Position wiederholt anzuordnen, z.B. wenn die Nachweisschritte der Kontaktierung mit unterschiedlichen Antikörperkonjugaten in einer anderen Position des Trägersubstrats durchgeführt werden.

Nachdem an die Probe gebundenes Antikörperkonjugat, bzw. dessen Fluorochromanteil optisch detektiert wurde, kann der Fluorochromanteil unwirksam gemacht bzw. inaktiviert werden, erfindungsgemäß bevorzugt durch Hydrolyse des Oligonukleotidlinkers oder durch Kontaktierung der Probe mit einem Quencher, der mit einem zum Oligonukleotidlinker abschnittsweise revers komplementären Oligonukleotid verbunden ist. Zusätzlich oder alternativ zu dem am Oligonukleotidlinker gebundenen Fluorochromanteil kann ein hybridisierbares Fluorochrom mit dem Antikörperkonjugat kontaktiert werden, was zur spezifischen Hybridisierung eines Fluorochroms an das Antikörperkonjugat führt.

Für die Zwecke der Erfindung sind Oligonukleotide und deren Abschnitte insbesondere dann hybridisierbar, wenn sie unter zellphysiologischen Bedingungen, z.B. in einem zellphysiologischen Puffermedium, vorzugsweise bei 20-37°C , spezifisch ein Dimer ausbilden. Insbesondere bezeichnet ein zu einem Quencherabschnitt bzw. Fluorochromabschnitt des Oligonukleotidlinkers revers komplementäres Oligonukleotid, das mit dem Quencher bzw. Fluorochrom verbunden ist, ein Oligonukleotid, das in den während des Nachweisverfahrens verwendeten wässrigen Zusammensetzungen, beispielsweise PBS (phosphatgepufferte Salzlösung), HEPES-Puffer, Tris-Puffer, vorzugsweise bei pH 6,5 bis 7,5 bei Salzkonzentrationen im Bereich von 20 bis 800 mM der Summe ein- und zweiwertiger Ionen mit dem Oligonukleotidlinker ein doppelsträngiges Nukleinsäurehybrid ausbildet, das insbesondere sequenzspezifisch ist. Dies sind insbesondere Oligonukleotide mit einer zu einem Abschnitt des Oligonukleotidlinkers revers komplementären Sequenz, z.B. mit einer Identität von mindestens 80%, bevorzugt mindestens 90%, besonders bevorzugt mindestens 95 bis 99% zur revers komplementären Nukleinsäuresequenz.

In Varianten, in der der Oligonukleotidlinker in einem Abschnitt doppelsträngig ist, in dem ein Oligonukleotidlinkerabschnitt und ein mit dem Fluorochromanteil verbundener Oligonukleotidabschnitt hybridisierbar bzw. hybridisiert sind, weisen der Fluorochromabschnitt und der zweite Oligonukleotidlinkerabschnitt vorzugsweise zueinander revers komplementäre Nukleinsäuresequenzen auf, die in den während des Nachweisverfahrens verwendeten wässrigen Zusammensetzungen ein Nukleinsäurehybrid ausbilden, insbesondere mit einer revers komplementären Sequenzidentität von mindestens 80%, bevorzugt mindestens 90%, besonders bevorzugt mindestens 95 bis 99%.

Der Oligonukleotidlinker, vorzugsweise auch das am Quencher gebundene Oligonukleotid und der zweite Oligonukleotidlinkerabschnitt des Fluorochroms weisen vorzugsweise Nukleinsäuresequenzen auf, die nicht in der DNA und/oder RNA der zu analysierenden Probe, z.B. der zu analysierenden tierischen Zelle vorkommen, insbesondere eine Sequenzabweichung von mindestens 5%, bevorzugter mindestens 10%, noch bevorzugter mindestens 20% haben. Bevorzugte Längen des Oligonukleotidlinkers, des am Quencher gebundenen Oligonukleotids und des zweiten Oligonukleotidlinkerabschnitts des Fluorochroms betragen 10 bis 150 nt (Nukleotide), vorzugsweise 15 bis 100 nt, bevorzugter 20 bis 50 nt, von denen mindestens 10, bevorzugter 30 nt, noch bevorzugter alle komplementär zueinander sind.

Ein geeignetes Fluorochrom kann ausgewählt werden aus Cy3, PE, FITC, APC, Alexa-Farbstoffen und Atto-Farbstoffen; ein geeigneter Quencher kann auf das Fluorochrom abgestimmt werden, z.B. aus der Gruppe, die Dabcyl, Dabsyl, Dark-Hole-Quencher, Black-Berry-Quencher und QSY-Farbstoffe umfasst.

### Genaue Beschreibung der Erfindung

Die Erfindung wird nun genauer anhand von Beispielen mit Bezug auf die Figuren beschrieben, in denen
- Figur 1 eine schematische Darstellung des Detektionsverfahrens der ersten Ausführungsform zeigt,
- Figur 2 eine schematische Darstellung des Detektionsverfahrens in einer Variante der zweiten Ausführungsform zeigt,
- Figur 3 eine schematische Darstellung des Detektionsverfahrens der ersten Ausführungsform in einer Variante der ersten Ausführungsform zeigt,
- Figur 4a) bis d) immobilisierte menschliche Leukozyten nach Inkubation mit erfindungsgemäßem Antikörper im mikroskopischen Bild unter Anregung der Fluoreszenz in den Schritten des Detektionsverfahrens, und
- Figur 5a) bis d) aus einer Lösung immobilisierte zellfreie Antigene nach Inkubation mit erfindungsgemäßem Antikörper im mikroskopischen Bild unter Anregung der Fluoreszenz in den Schritten des Detektionsverfahrens.
- Figur 6 mikroskopische Aufnahmen A) einer biologischen Probe mit einem erfindungsgemäßen Antikörper und B) eine mikroskopische Aufnahme der selben Probe, jedoch nach Hydrolyse der unter A) verwendeten Antikörperkonjugats und Inkubation mit einem zweiten Antikörperkonjugat mit anderer Antigenspezifität an der fixierten Probe zeigt, unter C) DZ - Ergebnisse der unbehandelten Zellprobe, unter D) DZ - Ergebnisse eines Aliquots der Probe nach Inkubation mit einem erfindungsgemäßen Antikörper, und E) DZ - Ergebnisse eines Aliquots der unter D) analysierten Probe nach Hydrolyse des Linkers.

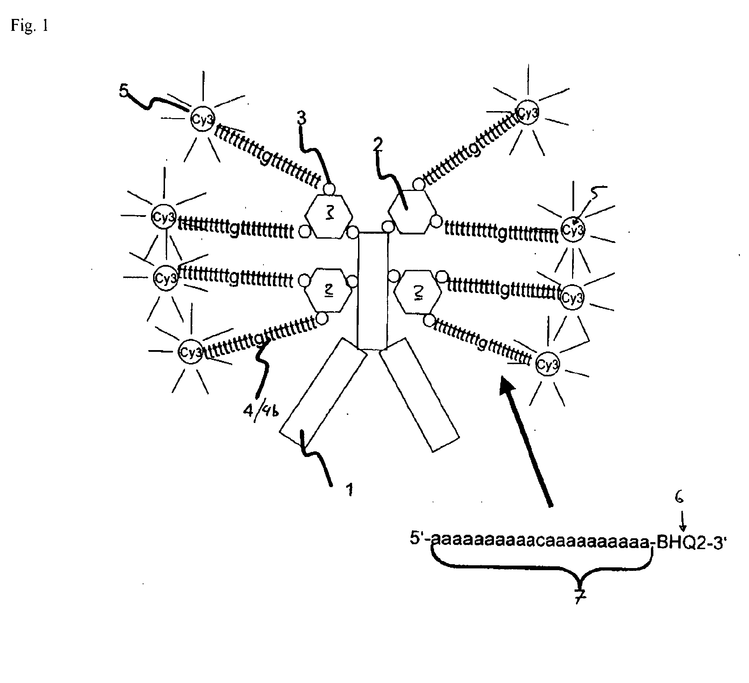

Figur 1 zeigt schematisch den Aufbau eines erfindungsgemäßen Konjugats der ersten Ausführungsform, das als erstes Reagenz eines Testkits aus dem Bindeanteil 1, dem Fluorochromanteil 5 und dem diese verbindenden Oligonukleotidlinker 4 besteht. Der Fluorochromanteil 5, hier Cy3, ist kovalent, z.B. über bifunktionale Verbindungen, an das erste Ende des Oligonukleotidlinkers 4 gebunden. Der Bindeanteil 1 ist schematisch in Form eines IgG-Antikörpers gezeigt, an dem mittels eines kovalent gebundenen ersten Kopplungsreagenzes 2, z.B. Streptavidin, und mittels eines am ersten Kopplungsreagenz 2 gebundenen zweiten Kopplungsreagenzes 3, z.B. Biotin, das zweite Ende des Oligonukleotidlinkers 4 gebunden ist. Bei Einstrahlung von Licht (hv) mit einer Anregungswellenlänge für das Fluorochrom 5 wird von dem Fluorochromanteil 5 Strahlung emittiert.

Zur Bindung des zweiten Kopplungsreagenzes 3 kann der Oligonukleotidlinker 4 terminal eine Biotin-Akzeptor-Domäne (BAD) aufweisen. Alternativ zur Bindung des Oligonukleotidlinkers 4 an den Bindeanteil 1 mittels erstem und zweiten Kopplungsreagenz kann die Bindung kovalent sein, z.B. durch Derivatisierung des Bindeanteils 1 und/oder des Oligonukleotidlinkers 4, z.B. zur Bildung einer Ester-, Amid- oder Thiobindung. Die Bindung des Fluorochroms 5 an den Oligonukleotidlinker 4 ist vorzugsweise kovalent.

Der Oligonukleotidlinker 4 kann die Nukleinsäuresequenz TTT TTT TTT TGT TTT TTT TT (Seq.-ID. Nr. 1) von 5' nach 3' aufweisen, die als Quencherabschnitt 4b durch Kontaktierung bzw. Hybridisierung mit dem Oligonukleotid 7, das mit einem Quencher 6 verbunden ist, den Quencher 6 in die Nähe des Fluorochromanteils 5 anordnet. Dadurch wird der Fluorochromanteil 5 optisch unwirksam gemacht. Das mit dem Quencherabschnitt 4b des Oligonukleotidlinkers 4 hybridisierbare Oligonukleotid 7 ist vorzugsweise abschnittsweise zum Quencherabschnitt 4b revers komplementär, beispielsweise mit der Nukleinsäuresequenz AAA AAA AAA ACA AAA AAA AAA (Seq.-ID Nr. 2, von 5' nach 3').

Zur Positionierung des Quenchers 6 in die Nähe, insbesondere innerhalb des Förster-Radius des Fluorochroms 5, ist der Quencher 6 vorzugsweise an das Ende des Oligonukleotids 7 gebunden, das bei Hybridbildung mit dem Oligonukleotidlinker 4 dem Fluorochrom 5 benachbart ist.

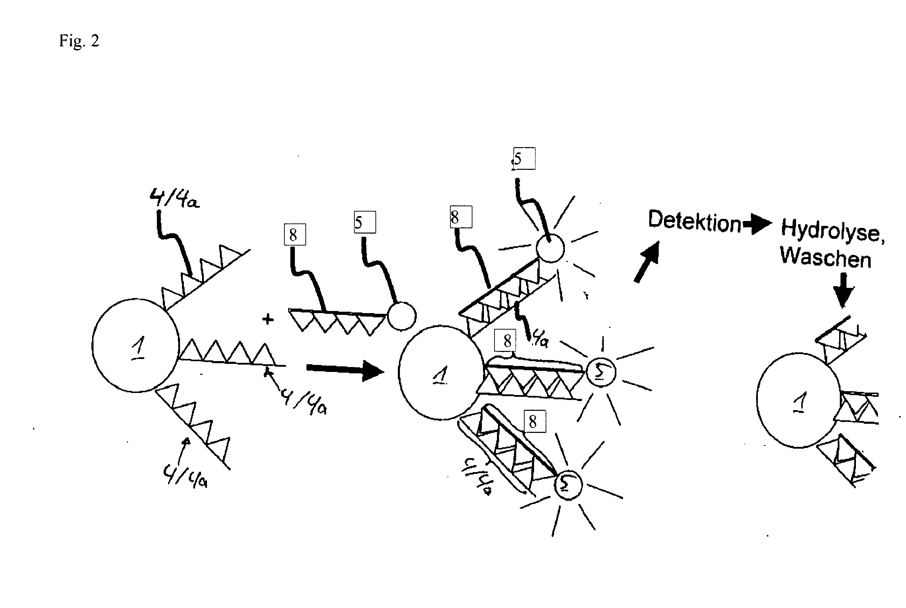

Für das Analyseverfahren kann eine Probe mit dem ersten Reagenz, das aus Bindeanteil 1, Fluorochromanteil 5 und dem mit seinem ersten Ende am Fluorochromanteil 5 angebundenen Oligonukleotidlinker 4 besteht, kontaktiert werden. Nach Waschen kann die spezifische Bindung des Bindeanteils 1 an die Probe durch Detektion des Fluorochromanteils analysiert werden. Der Oligonukleotidlinker 4 weist einen Quencherabschnitt 4b auf, zu dem ein Oligonukleotid 7 hybridisierbar ist, das mit dem Quencher 5 verbunden ist. Durch Kontaktieren des an der Probe gebundenen ersten Reagenzes mit einem zweiten Reagenz, das einen an einem Oligonukleotid 7 gebundenen Quencher 6 aufweist, wird der Fluorochromanteil 5 unwirksam gemacht. Die nachfolgende Kontaktierung der Probe mit einem weiteren ersten Reagenz ermöglicht die Detektion von dessen Fluorochromanteil ohne Beeinträchtigung durch das zuvor mit der Probe kontaktierte erste Reagenz, dessen Fluorochrom durch Anordnung des Quenchers 6 in dessen Nähe unwirksam gemacht ist. Als Alternative zum Unwirksammachen durch spezifische Anordnung eines Quenchers 6 am ersten Reagenz kann dessen Fluorochromanteil 5 durch Hydrolyse des Oligonukleotidlinkers 4 und Waschen effektiv unwirksam gemacht werden, wie für die zweite Ausführungsform in Figur 2 schematisch dargestellt ist.

Figur 2 zeigt schematisch den Aufbau eines erfindungsgemäßen Konjugats der zweiten Ausführungsform, das aus dem Bindeanteil 1, hier in Form eines Rezeptorliganden gezeigt, und daran mittels eines kovalent am Bindeanteil 1 gebundenen Oligonukleotidlinkers 4 gebundenen Fluorochroms 5 besteht. Der Oligonukleotidlinker 4 ist in dem Abschnitt doppelsträngig, in dem der am Fluorochrom 5 gebundene zweite Oligonukleotidlinkerabschnitt 8 mit dem hybridisierbaren ersten Oligonukleotidlinkerabschnitt 4a hybridisiert ist. Vorzugsweise sind die Bindungen zwischen Bindeanteil 1 und erstem Oligonukleotidlinkerabschnitt 4a, sowie zwischen zweitem Oligonukleotidlinkerabschnitt 8 und Fluorochrom 5 durch Assoziation jeweils an einem Bestandteil kovalent gebundener erster und zweiter Kopplungsreagenzien entsprechend Figur 1 gebildet, oder kovalent.

Da die zweite Ausführungsform auch in dieser Variante des abschnittsweise doppelsträngigen Oligonukleotidlinkers 4 einen durch eine Endonuklease hydrolysierbaren Oligonukleotidlinker 4 aufweist, ist hier eine Endonuklease als zweites Reagenz im Testkit enthalten. Entsprechend weist hier der Oligonukleotidlinker 4 optional keinen Quencherabschnitt 4b auf.

Im Analyseverfahren kann die Probe mit einem ersten Anteil des ersten Reagenzes kontaktiert werden, der aus Bindeanteil mit gebundenem Oligonukleotidlinker 4 mit einem einen Fluorochromabschnitt enthaltenden ersten Oligonukleotidlinkerabschnitt 4a besteht, und gleichzeitig oder anschließend mit einem zweiten Anteil kontaktiert werden, der den an den ersten Oligonukleotidlinkerabschnitt 4a hybridisierbaren bzw. hybridisierten zweiten Oligonukleotidlinkerabschnitt 8 gebundenen Fluorochromanteil 5 enthält. Nach Entfernung ungebundener Anteile von der Probe durch Waschen kann spezifisch gebundener Bindeanteil 1 durch Detektion des daran über den abschnittsweise doppelsträngigen Oligonukleotidlinker 4 gebundenen Fluorochromanteil 5 nachgewiesen werden. Zum Unwirksammachen des Fluorochromanteils 5 kann der Oligonukleotidlinker 4 durch Kontaktieren mit einer Endonuklease (nicht dargestellt) und anschließendes Waschen von dem Bindeanteil 1 entfernt und dadurch effektiv unwirksam gemacht werden.

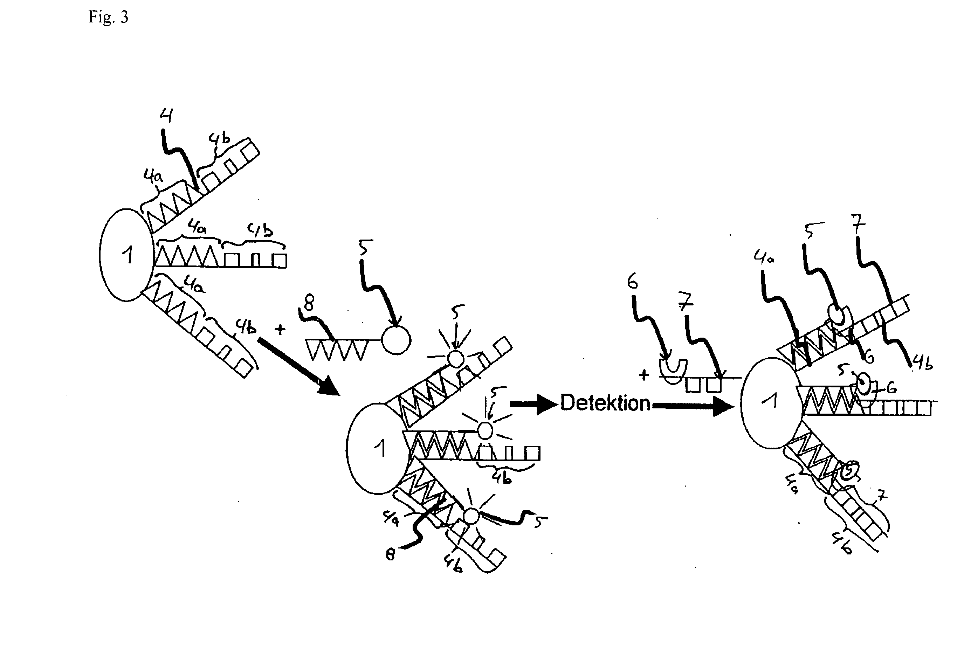

Figur 3 zeigt schematisch den Aufbau eines erfindungsgemäßen Konjugats der ersten Ausführungsform in der Variante, dass der Oligonukleotidlinker 4 abschnittsweise doppelsträngig ist. Der Bindeanteil 1 ist an den Oligonukleotidlinker 4 gebunden, der einen ersten Oligonukleotidlinkerabschnitt 4a mit Fluorochromabschnitt aufweist, an den der an den Fluorochromanteil 5 gebundene zweite Oligonukleotidlinkerabschnitt 8 hybridisierbar ist. Der Oligonuklcotidlinker 4 weist entsprechend den ersten Oligonukleotidlinkerabschnitt 4a auf, zu dem der zweite Oligonukleotidlinkerabschnitt 8, an den das Fluorochrom 5 gebunden ist, abschnittsweise hybridisierbar, z.B. revers komplementär ist. Angrenzend an den ersten Oligonukleotidabschnitt 4a weist der Oligonukleotidlinker 4 einen Quencherabschnitt 4b auf, zu dem das Oligonukleotid 7 hybridisierbar, z.B. revers komplementär ist, welches an den Quencher 6 gebunden ist. Zur Positionierung des Fluorochroms 5 in die Nähe des Quenchers 6 ist das Fluorochrom 5 vorzugsweise an das Ende des zweiten Oligonukleotidlinkerabschnitts 8 gebunden, das bei Hybridisierung mit dem ersten Oligonukleotidlinkerabschnitt 4a an den Quencherabschnitt 4b des Oligonukleotidlinkers 4 angrenzt, mit welchem das Oligonukleotid 7 hybridisiert, das den Quencher 6 trägt. Zur Positionierung des Quenchers 6 in die Nähe, insbesondere innerhalb des Förster-Radius des Fluorochroms 5, ist der Quencher 6 vorzugsweise an das Ende des Oligonukleotids 7 gebunden, das bei Hybridbildung mit dem Oligonukleotidlinker 4, bzw. dessen Quencherabschnitt 4b, an den ersten Oligonukleotidlinkerabschnitt 4a des Oligonukleotidlinkers 4 angrenzt, an den der zweite Oligonukleotidlinkerabschnitt 8 des Fluorochroms 5 hybridisierbar ist.

Bei Hybridisierung des zweiten Oligonukleotidlinkerabschnitts 8, der mit dem Fluorochrom 5 verbunden ist, mit dem ersten Oligonukleotidlinkerabschnitt 4 wird der Bindeanteil 1 mit dem Fluorochrom 5 verbunden, so dass bei Einstrahlung von Licht hv mit Anregungswellenlänge Fluoreszenz emittiert wird. Diese Emission wird erfindungsgemäß zur Detektion des Konjugats gemessen.

Der Zusatz von hybridisierbarem Quencher 6, der mit einem zu einem Quencherabschnitt 4b des Oligonukleotidlinkers 4 komplementären Oligonukleotid 7 verbunden ist, führt durch Positionierung des Quenchers 6 in die Nähe des Fluorochroms 5 zum Unwirksammachen des Fluorochroms 5.

Wie in Figuren 1 bis 3 gezeigt, können erfindungsgemäße Konjugate ein oder mehr Oligonukleotidlinker 4 aufweisen, die an den Bindeanteil 1 gebunden sind.

### Beispiel 1: Herstellung eines Antikörper-Fluorochrom-Konjugats mit Oligonukleotidlinker

Als Beispiel für ein erfindungsgemäßes Konjugat der ersten Ausführungsform wurde ein biotinylierter anti-Maus-CD4-Antikörper (Klon Sk1.5, erhältlich von BD Bioscience) mit Streptavidin inkubiert und das entstandene Streptavidin-Antikörperkonjugat wurde aufgereinigt. Als Oligonukleotidlinker wurde synthetisch hergestellte einsträngige DNA der Seq.-ID Nr. 1 verwendet, an deren 3'-Ende Biotin und an deren 5'-Ende das Fluorophor Cy3 (erhältlich von Molecular Probes Inc.) gekoppelt war. Zur Bindung des Oligonukleotidlinkers an den als Bindeanteil eingesetzten Antikörper wurde das biotinylierte Oligonukleotid mit dem Streptavidin-gekoppelten Antikörper inkubiert und anschließend gelchromatographisch aufgereinigt. Die Bindung zwischen Streptavidin und Biotin ergab ein Konjugat der Struktur Antikörper-Biotin-Streptavidin-Biotin-Oligonukleotidlinker-Fluorochrom.

### Beispiel 2: Detektion eines Antigens auf der Zelloberfläche menschlicher Zellen

Als Beispiel für eine biologische Probe wurden menschliche Leukozyten aus peripherem Blut isoliert und auf einem Objektträger aus Glas mit einer zelladhäsiven Beschichtung (Polylysin, erhältlich von Karl Roth GmbH) immobilisiert. Der Objektträger wies zwei parallele beabstandete Vorsprünge als Abstandshalter auf, die ein zum Objektträger paralleles Deckglas trugen. Die jeweils an den Enden des Objektträgers entstandenen Öffnungen wurden als Einlass- bzw. Auslassöffnung zur Zu- und Abführung von Lösungen verwendet.

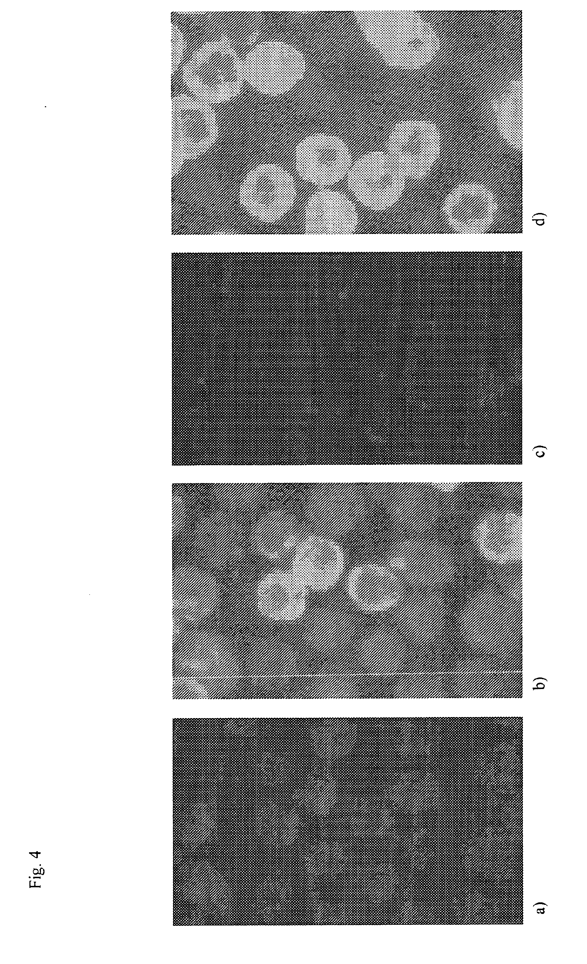

Nach Entfernen ungebundener Bestandteile durch Waschen mit physiologischer phosphatgepufferter Salzlösung (PBS, pH 7,4) wurden die Zellen mikroskopisch (Axioplan 2e Mikroskop, Zeiss, mit motorischer Objekttischverstellung und Fokussierung, Quecksilberlampe HBO 100 zur Anregung, Filter für PE oder FITC, Plan-Neofluar 16x/0,50 Immersionsobjektiv und CCD Kamera Axiocam MRm zur Aufzeichnung) unter Einstrahlung einer Anregungswellenlänge analysiert, um die Hintergrund-Fluoreszenz zu bestimmen. Das erhaltene mikroskopische Bild ist in Figur 4 a) gezeigt, in dem lichtmikroskopisch identifizierte Zellen durch eingefügte Kreise markiert sind. Anschließend wurde Antikörperkonjugat nach Beispiel 1 zugegeben, das als Antikörperanteil anti-CD4-Antikörper enthielt. Figur 4 b) zeigt die selbe Stelle wie Figur 4 a) bei Fluoreszenzdetektion des vom Fluorochrom Cy3 emittierten Lichts. Diese Aufnahme zeigt, dass erfindungsgemäße Konjugate zum spezifischen Nachweis von Zellantigenen geeignet sind, da die CD4-positiven Zellen selektiv markiert sind.

Anschließend wurde ein mit einem Quencher verbundenes Oligonukleotid durch die Einlaßöffnung zu den Zellen gegeben, nämlich das zu Seq.-ID Nr. 1 abschnittsweise revers komplementäre Oligonukleotid Seq.-ID Nr. 2, an dessen 3'-Ende der Quencher BHQ2 (Black-Hole-Quencher, erhältlich von BioSearch, Novato, USA) gebunden war. Nach Inkubation für 1 min bein Raumtemperatur wurde zum Waschen 100 µL PBS an der Einlassöffnung zugegeben und etwas dasselbe Volumen an der Auslassöffnung abgenommen. Eine erneute fluoreszenzmikroskopische Aufnahme der selben Stelle ist in Figur 4c) gezeigt. Dieses Ergebnis macht deutlich, dass die optische Aktivität des Fluorochroms des erfindungsgemäßen Konjugats selektiv unwirksam gemacht werden kann. Alternativ zum Zusatz des Oligonukleotid-tragenden Quenchers konnte zum Unwirksammachen des Fluorochromanteils DNase zugesetzt werden, gefolgt von Waschen mit ca. 100 bis 200 µL PBS, um den Oligonukleotidlinker des Konjugats zu hydrolysieren und das Fluorochrom zu entfernen.

Anschließend wurden dieselben immobilisierten Zellen mit einem Antikörperkonjugat kontaktiert, das entsprechend Beispiel 1 hergestellt war, jedoch mit einem anti-Maus-CD19-Antikörper. Die fluoreszenzmikroskopische Aufnahme ist in Figur 4d) gezeigt, die deutlich macht, dass erfindungsgemäße Konjugate zur aufeinanderfolgenden Detektion von Antigenen geeignet sind, jeweils mit spezifischen Unwirksammachen des Fluorochromanteils eines Konjugats vor Kontaktierung der Probe mit einem weiteren Konjugat. Auch die Fluoreszenz des anti-CD19-Konjugats konnte durch Zusatz des selben Quenchers, der das zum Oligonukleotidlinker revers komplementäre Oligonukleotid trug, oder durch Zusatz von DNase unwirksam gemacht, d.h. entfernt werden.

### Beispiel 3: Nacheinander folgende Detektion löslicher Antigene nach Immobilisierung auf einem Trägersubstrat

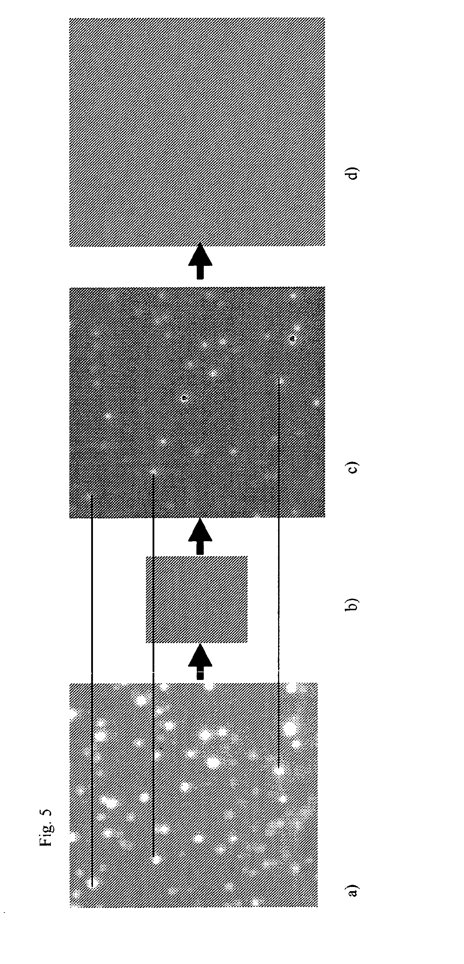

Erfindungsgemäße Konjugate wurden zur Analyse zellfreier Analyten verwendet, beispielhaft zur Detektion von oberflächlich gebundenem IgG2a. Auf einem mit Polylysin beschichteten Objektträger aus Glas, wie in Beispiel 2 verwendet, wurde Phycoerythrin-markiertes Ratten-Immunglobulin Ig2Ga kappa binden gelassen. Anschließend wurden freie Bindungsstellen des Objektträgers mit Rinderserumalbumin (3% in PBS) gesättigt. Eine fluoreszenzmikroskopische Aufnahme ist in Figur 5a) gezeigt, in der die Fluoreszenz einzelner Moleküle sichtbar ist. Die Position des markierten IgG2a wurde festgestellt und in Bezug zum Objektträger gespeichert.

Zur Auslöschung der Eigenfluoreszenz des Phycoerythrin-markierten Ratten-Immunglobulins wurde mit der spezifischen Anregungswellenlänge (488nm) bestrahlt. Die Einzelmolekül- und fluoreszenzmikroskopische Aufnahme in Figur 5b) zeigt, dass die Fluoreszenzaktivität des Phycorerythrins dadurch ausgelöscht wurde.

Anschließend wurde ein Antikörperkonjugat zugegeben, das entsprechend Beispiel 1 hergestellt wurde, jedoch mit einem anti-IgG2a-Antikörper. Ungebundenes Konjugat wurde durch Waschen mit PBS entfernt. Die anschließende fluoreszenzmikroskopische Aufnahme von Figur 5c) zeigt, dass dieselben Positionen wie in Figur 5a) von dem erfindungsgemäßen Konjugat markiert wurden, also diese Konjugate auch zur Identifikation zellfreier Analyten geeignet sind.

Durch nachfolgende Inkubation mit dem mit einem revers komplementären Oligonukleotid verbundenen Quencher aus Beispiel 2 führte zum Unwirksammachen der für den Analyten spezifischen Fluoreszenz des erfindungsgemäßen Konjugats. Eine fluoreszenzmikroskopische Aufnahme ist in Figur 5d) gezeigt, aus der die Auslöschung der Fluoreszenz der Konjugate deutlich wird. Als Alternative zum Quencher konnte DNase zugesetzt werden und die immobilisierten Analyten gewaschen werden, um die Fluoreszenz des Konjugats unwirksam zu machen.

### Beispiel 4: Hydrolyse eines erfindungsgemäßen Antikörperkoniugats an immobilisierten menschlichen Zellen und an suspendierten menschlichen Zellen (DZ)

Es konnte gezeigt werden, dass der Fluorochromanteil eines erfindungsgemäßen Antikörperkonjugats, das nach Beispiel 1 erhältlich ist und dessen Antikörperanteil ein anti-CD4-Antikörper und dessen Fluorochromanteil Cy3 war, die mit einem Oligonukleotidlinker (Seq.-ID Nr. 1) verbunden waren, an lebenden, immobilisierten Immunzellen und an suspendierten lebenden Immunzellen unwirksam gemacht werden konnte.

Entsprechend Beispiel 2 wurden immobilisierte menschliche Immunzellen mit dem Antikörperkonjugat inkubiert; die mikroskopische Aufnahme einschließlich der detektierten

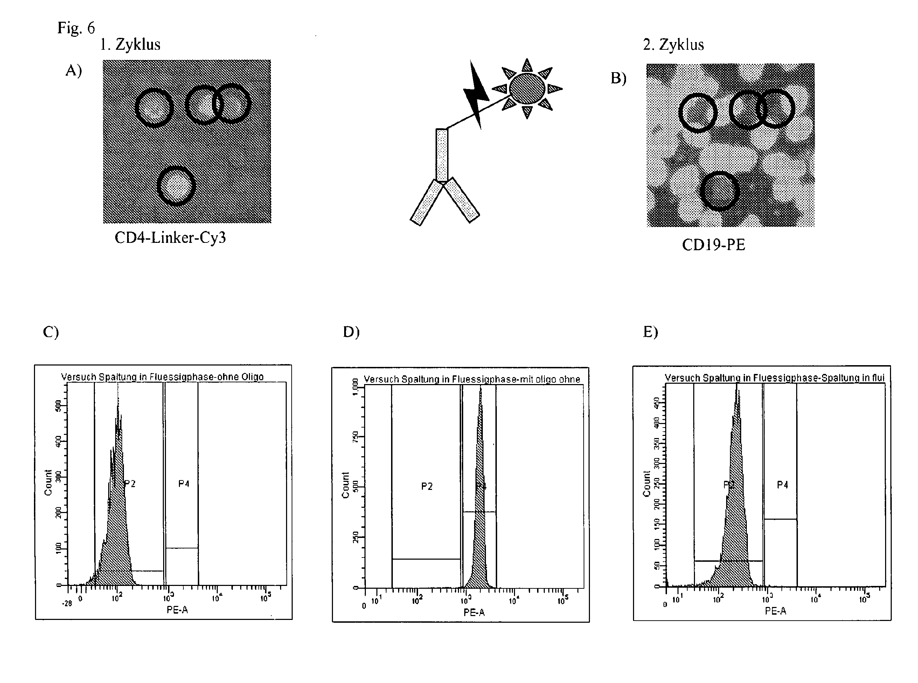

Fluoreszenz ist in Figur 6 A) gezeigt. Anschließend wurde die Probe durch Kontaktierung mit DNase behandelt, um den Fluorochromanteil abzutrennen. Zur Entfernung ungebundenen Fluorochroms wurde die immobilisierte Probe gewaschen. Alternativ wurde der in Beispiel 2 verwendete hybridisierbare Quencher mit zum Quencherabschnitt des Oligonukleotidlinkers revers komplementären Oligonukleotid zugegeben, was ebenfalls zum Unwirksammachen des Fluorochroms führte.

Anschließend wurde die Probe mit einem Antikörperkonjugat mit dem selben Oligonukleotidlinker, jedoch mit einem anti-CD19-Antikörperanteil und PE als Fluorochromanteil inkubiert; das mikroskopische Abbild detektierter Emissionen ist in Figur 5 B) gezeigt und macht deutlich, dass die mit dem anti-CD4-Antikörperkonjugat detektierten Zellen (jeweils durch manuell eingefügte Kreise gekennzeichnet) in der Analyse mit anti-CD19-Antikörperkonjugat kein Fluoreszenzsignal ergaben.

Zum Nachweis des effektiven Unwirksammachens des Fluorochromanteils eines Antikörperkonjugats durch Abtrennen des Fluorochromanteils wurde ein Aliquot von Zellen ohne Antikörperkonjugat, ein Aliquot der Zellen nach Inkubation mit Antikörperkonjugat (anti-CD4-Antikörper-Oligonukleotidlinker-Cy3), und davon ein Aliquot nach Hydrolyse des Linkers durch Inkubation mit DNase per DZ analysiert. Die Ergebnisse sind in Figuren 7C) für nicht mit Antikörperkonjugat inkubierte Zellen, D) mit anti-CD4 - Antikörperkonjugat inkubierte Zellen und E) nach Hydrolyse des Linkers von Zellen, die mit anti-CD4-Antikörperkonjugat inkubiert waren, gezeigt. Die DZ-Analysen machen deutlich, dass das erfindungsgemäße Antikörperkonjugat den Oberflächenmarker effektiv nachweisen konnte und das an die Zellen gebundene Antikörperkonjugat ein detektierbares Signal emittierter Strahlung erzeugt, wobei die Hydrolyse des Oligonukleotidlinkers mit anschließendem Waschen zum vollständigen Unwirksammachen des Fluorochromanteils führte. Entsprechende Ergebnisse wurden auch durch Zusatz des hybridisierbaren Quenchers mit einem zum Quencherabschnitt des Oligonukleotidlinkers revers komplementären Oligonukleotid erhalten.

### Beispiel 5: Verwendung der Konjugate zur iterativen Zellsortierung per DZ

Milzzellen der Maus wurden in Suspension in PBS mit einem entsprechend Beispiel 1 hergestellten anti-CD3-Antikörperkonjugat für 10 min bei Raumtemperatur inkubiert. Per Durchflusszytometrie (FACXAria, BD Biosciences) werden fluoreszenzmarkierte Zellen, hier T-Helferlymphozyten, von den übrigen Zellen abgetrennt. Die T-Helferlymphozyten werden dann für 10 min bei Raumtemperatur in PBS mit dem in Beispiel 2 verwendeten Quencher mit Oligonukleotid inkubiert. Die anschließende Analyse per DZ zeigte, dass der Fluorochrom unwirksam gemacht war. Nach Abtrennen des Quenchers konnten die T-Heiferlymphozyten mit einem zweiten Antikörperkonjugat analysiert und sortiert werden, das einen anderen Antikörperanteil aufwies, jedoch den gleichen Oligonukleotidlinker und dasselbe Fluorochrom.

### Bezugszeichenliste:

1 Bindeanteil
2 erstes Kopplungsreagenz
3 zweites Kopplungsreagenz
4 Oligonukleotidlinker
4a Fluorochromabschnitt
4b Quencherabschnitt
5 Fluorochrom
6 Quencher
7 zum Quencherabschnitt 4b des Oligonukleotidlinkers 4 revers komplementäres Oligonukleotid
8 mit dem Fluorochromabschnitt des Oligonukleotidlinkers hybridisierbarer zweiter Oligonukleotidlinkerabschnitt

## Patentansprüche

1. Zusammenstellung von Reagenzien zum Nachweis von Analyten in Proben, umfassend ein erstes Reagenz, das ein Nachweiskonjugat mit einem für einen Analyten spezifischen Bindeanteil (1), einem mit dem Bindeanteil (1) verbundenen Fluorochromanteil (5) und einem mit seinem ersten Ende mit dem Fluorochromanteil verbundenen Oligonukleotidlinker (4) aufweist, der zumindest abschnittsweise ein Oligonukleotid ist, das in seiner Nukleinsäuresequenz einen Quencherabschnitt (4b) aufweist, und
ein zweites Reagenz, das einen Quencher (6) aufweist, der mit dem Ende eines mit dem Quencherabschnitt (4b) hybridisierbaren Oligonukleotids (7) verbunden ist, das bei Hybridisierung mit dem Oligonukleotidlinker (4) an dessen ersten Ende liegt.

2. Zusammenstellung nach Anspruch 1, **dadurch gekennzeichnet, dass** der Oligonukleotidlinker (4) mit seinem zweiten Ende, das dem ersten Ende gegenüberliegt, mit dem Bindeanteil (1) verbunden ist.

3. Zusammenstellung nach einem der voranstehenden Ansprüche, **dadurch gekennzeichnet, dass** der Oligonukleotidlinker (4) einen ersten Oligonukleotidlinkerabschnitt (4a) aufweist, der an den Bindeanteil (1) gebunden ist, wobei der erste Oligonukleotidlinkerabschnitt (4a) in seiner Nukleinsäuresequenz einen Fluorochromabschnitt (4a) aufweist, und dass der Fluorochromanteil (5) mit einem mit dem Fluorochromabschnitt (4a) hybridisierbaren zweiten Oligonukleotidlinkerabschnitt (8) verbunden ist.

4. Zusammenstellung nach Anspruch 3, **dadurch gekennzeichnet, dass** der zweite Oligonukleotidlinkerabschnitt (8), der mit dem Fluorochromanteil (5) verbunden ist, den Quencherabschnitt (4b) aufweist.

5. Zusammenstellung nach Anspruch 4, **dadurch gekennzeichnet, dass** zumindest zwei Nachweiskonjugate enthalten sind, die jeweils Fluorochromabschnitte (4a) mit identischen oder ähnlichen Nukleotidsequenzen aufweisen, an die unter physiologischen Bedingungen der selbe zweite Oligonukleotidlinkerabschnitt (8) hybridisierbar ist.

6. Zusammenstellung nach Anspruch 4, **dadurch gekennzeichnet, dass** zumindest zwei Nachweiskonjugate enthalten sind, die jeweils Fluorochromabschnitte (4a) mit identischen oder ähnlichen Nukleotidsequenzen aufweisen, an die unter physiologischen Bedingungen der selbe zweite Oligonukleotidlinkerabschnitt (8) hybridisierbar ist.

7. Zusammenstellung nach einem der voranstehenden Ansprüche, **dadurch gekennzeichnet, dass** zumindest zwei Nachweiskonjugate enthalten sind, die jeweils Quencherabschnitte (4b) mit einzigartigen Nukleotidsequenzen aufweisen.

8. Zusammenstellung nach einem der Ansprüche 1 bis 6, **dadurch gekennzeichnet, dass** zumindest zwei Nachweiskonjugate enthalten sind, die jeweils Quencherabschnitte (4b) mit identischen oder ähnlichen Nukleotidsequenzen aufweisen, an die unter physiologischen Bedingungen das selbe mit dem Quencher (6) verbundene Oligonukleotid (7) hybridisierbar ist.

9. Zusammenstellung nach einem der voranstehenden Ansprüche, **dadurch gekennzeichnet, dass** das zweite Reagenz kein mit dem Oligonukleotid (7) verbundener Quencher (6) ist und optional der Oligonukleotidlinker (4) keinen Quencherabschnitt (4b) aufweist, und dass das zweite Reagenz der Zusammenstellung eine Nuklease ist.

10. Zusammenstellung nach Anspruch 9, **dadurch gekennzeichnet, dass** der Oligonukleotidlinker (4) eine Erkennungssequenz für eine Restriktase aufweist und die Nuklease eine für die Erkennungssequenz spezifische Restriktase ist.

11. Zusammenstellung nach einem der voranstehenden Ansprüche, **dadurch gekennzeichnet, dass** unabhängig voneinander der Oligonukleotidlinker (4) und/oder das mit dem Quencher (6) verbundene Oligonukleotid (7) eine einsträngige Oligoribonukleinsäure (RNA), Oligodesoxyribonukleinsäure (DNA) oder Oligo-Peptidnukleinsäure (PNA) ist.

12. Zusammenstellung nach einem der Ansprüche 3 bis 11, **dadurch gekennzeichnet, dass** unabhängig voneinander der erste Oligonukleotidlinkerabschnitt (4a) des Oligonukleotidlinkers (4) und/oder der mit diesem hybridisierbare zweite Oligonukleotidlinkerabschnitt (8), der mit dem Fluorochromanteil (5) verbunden ist, eine einsträngige Oligoribonukleinsäure (RNA), Oligodesoxyribonukleinsäure (DNA) oder Oligo-Peptidnukleinsäure (PNA) ist.

13. Zusammenstellung nach einem der voranstehenden Ansprüche, **dadurch gekennzeichnet, dass** zwei oder mehr Oligonukleotidlinker (4) mit dem Bindeanteil (1) verbunden sind.

14. Zusammenstellung nach einem der voranstehenden Ansprüche, **dadurch gekennzeichnet, dass** der Bindeanteil (1) aus der Gruppe ausgewählt ist, die aus Antikörpern, Paratop bildenden Antikörperfragmenten, Lektinen, Aptameren, Peptiden, die für vorbestimmte MHCI oder MHCII spezifisch sind, MHC-Tetrameren, Biotin und Avidin besteht.

15. Verwendung einer Zusammenstellung nach einem der voranstehenden Ansprüche zur Analyse biologischer Proben, **gekennzeichnet durch** die aufeinander folgende Kontaktierung der Probe mit einem Nachweiskonjugat einer ersten Analytenspezifität, Kontaktierung der Probe mit dem zweiten Reagenz, und Kontaktierung der Probe mit einem Nachweiskonjugat einer zweiten Analytenspezifität.

16. Verfahren zur Analyse biologischer Proben, **gekennzeichnet durch** die Herstellung einer Zusammenstellung nach einem der Ansprüche 1 bis 14 in Kontakt mit der Probe.

17. Verfahren nach Anspruch 16, mit den Schritten
der Kontaktierung einer biologischen Probe mit einem Nachweiskonjugat, das einen Bindeanteil (1) mit einer ersten Spezifität für einen ersten Analyten, einen mit dem Bindeanteil (1) verbundenen Fluorochromanteil (5) und ein mit seinem ersten Ende mit dem Fluorochromanteil (5) verbundenen Linker aufweist,
des Detektierens von durch den Fluorochromanteil (5) emittierter Strahlung,
des Unwirksammachens des Fluorochromanteils (5),
**dadurch gekennzeichnet,**
**dass** der Linker ein Oligonukleotidlinker (4) ist, der zumindest abschnittsweise ein Oligonukleotid ist, das in seiner Nukleinsäuresequenz einen Quencherabschnitt (4b) aufweist, und
**dass** das Unwirksammachen des Fluorochromanteils (5) durch Kontaktieren des Nachweiskonjugats mit einem Quencher (6) erfolgt, der mit dem Ende eines mit dem Quencherabschnitt (4b) hybridisierbaren Oligonukleotids (8) verbunden ist, das bei Hybridisierung mit dem Oligonukleotidlinker (4) an dessen ersten Ende liegt.

18. Verfahren nach Anspruch 16 oder 17, **gekennzeichnet durch** die aufeinander folgende Kontaktierung der Probe mit einem Nachweiskonjugat einer ersten Analytenspezifität, Kontaktierung der Probe mit einem für das erste Nachweiskonjugat spezifischen Quencher und Kontaktierung der Probe mit einem Nachweiskonjugat einer zweiten Analytenspezifität.

19. Verfahren nach Anspruch 18, **dadurch gekennzeichnet, dass** der Fluorochromanteil (5) und der Oligonukleotidlinker (4) in nacheinander folgenden Kontaktierungen eingesetzter Nachweiskonjugate untereinander jeweils identisch sind und der Quencher (6) jeweils der gleiche mit gleichem Oligonukleotid (7) ist.

20. Verfahren nach Anspruch 16 oder 17, **dadurch gekennzeichnet, dass** das Unwirksammachen des Fluorochromanteils (5) an Stelle des Kontaktierens mit einem Quencher (6) durch Kontaktieren des Nachweiskonjugats mit einer Nuklease und anschließendes Entfernen ungebundener Bestandteile erfolgt.

21. Verfahren nach einem der Ansprüche 16 bis 19, **dadurch gekennzeichnet, dass** der Oligonukleotidlinker (4) eine ersten Oligonukleotidlinkerabschnitt (4a) aufweist, der an den Bindeanteil (1) gebunden ist und in seiner Nukleotidsequenz einen Fluorochromabschnitt (4a) aufweist und dass das Bindekonjugat durch Kontaktieren des Fluorochromabschnitts (4a) mit einem Fluorochromanteil (5) erzeugt wird, das mit einem mit dem Fluorochromabschnitt hybridisierbaren zweiten Oligonukleotidlinkerabschnitt (8) verbunden ist.

22. Verfahren nach Anspruch 21, **dadurch gekennzeichnet, dass** die Probe gleichzeitig mit zumindest zwei Nachweiskonjugaten kontaktiert wird, deren Fluorochromabschnitte (4a) jeweils einzigartig sind.

23. Verfahren nach einem der Ansprüche 16 bis 22, **dadurch gekennzeichnet, dass** die Probe gleichzeitig mit zumindest zwei Nachweiskonjugaten kontaktiert wird, deren Quencherabschnitte (4b) jeweils einzigartig sind.

24. Verfahren nach einem der Ansprüche 16 bis 23, **dadurch gekennzeichnet, dass** Analyten auf einem Trägersubstrat fixiert sind.

25. Verfahren nach Anspruch 24, **dadurch gekennzeichnet, dass** das Trägersubstrat Bestandteil eines Durchflusskanals ist, in dem die Analyten von wässrigen Zusammensetzungen umströmbar angeordnet sind.

## Claims

1. Combination of reagents for the detection of analytes in samples, comprising a first reagent having a detection conjugate having a binding portion (1) specific for an analyte, a fluorochrome portion (5) connected to the binding portion (1), and an oligonucleotide linker (4) connected by its first end to the fluorochrome portion, which oligonucleotide linker (4) at least in a section is an oligonucleotide having a quencher section (4b) within its nucleic acid sequence, and
a second reagent having a quencher (6) that is connected to the end of an oligonucleotide (7) which is hybridizeable to the quencher section (4b) and which upon hybridizing with the oligonucleotide linker (4) is located at the first end thereof.

2. Combination according to claim 1, **characterized in that** the oligonucleotide linker (4) is connected with its second end opposite the first end to the binding portion (1).

3. Combination according to one of the preceding claims, **characterized in that** the oligonucleotide linker (4) has a first oligonucleotide linker section (4a) that is bound to the binding portion (1), wherein the first oligonucleotide linker section (4a) in its nucleic acid sequence has a fluorochrome section (4a), and **in that** the fluorochrome portion (5) is connected to a second oligonucleotide linker section (8) hybridizeable to the fluorochrome section (4a).

4. Combination according to claim 3, **characterized in that** the second oligonucleotide linker section (8) that is connected to the fluorochrome portion (5) has the quencher section (4b).

5. Combination according to claim 4, **characterized in that** at least two detection conjugates are contained, each having fluorochrome sections (4a) having identical or similar nucleotide sequences to which the same second oligonucleotide linker section (8) is hybridizeable under physiological conditions.

6. Combination according to claim 4, **characterized in that** at least two detection conjugates are contained, each having fluorochrome sections (4a) having identical or similar nucleotide sequences to which the same second oligonucleotide linker section (8) is hybridizeable under physiological conditions.

7. Combination according to one of the preceding claims, **characterized in that** at least two detection conjugates are contained, each having quencher sections (4b) having unique nucleotide sequences.

8. Combination according to one of claims 1 to 6, **characterized in that** at least two detection conjugates are contained, each having quencher sections (4b) having identical or similar nucleotide sequences to which the same oligonucleotide (7) connected to the quencher (6) is hybridizeable under physiological conditions.

9. Combination according to one of the preceding claims, **characterized in that** the second reagent is no quencher (6) connected to the oligonucleotide (7) and optionally the oligonucleotide linker (4) has no quencher section (4b), and **in that** the second reagent of the combination is a nuclease.

10. Combination according to claim 9, **characterized in that** the oligonucleotide linker (4) has a recognition sequence for a restrictase and the nuclease is a restrictase specific for the recognition sequence.

11. Combination according to one of the preceding claims, **characterized in that** independent from one another, the oligonucleotide linker (4) and/or the oligonucleotide (7) connected to the quencher (6) is a single-stranded oligo ribonucleic acid (RNA), oligo deoxyribonucleic acid (DNA), or oligo peptide nucleic acid (PNA).

12. Combination according to one of the claims 3 to 11, **characterized in that** independent from one another, the first oligonucleotide linker section (4a) of the oligonucleotide linker (4) and/or the second oligonucleotide linker section (8) hybridizeable thereto which is connected to the fluorochrome portion (5), is a single-stranded oligo ribonucleic acid (RNA), oligo deoxyribonucleic acid (DNA), or oligo peptide nucleic acid (PNA).

13. Combination according to one of the preceding claims, **characterized in that** two or more oligonucleotide linkers (4) are connected to the binding portion (1).

14. Combination according to one of the preceding claims, **characterized in that** the binding portion (1) is selected from the group consisting of antibodies, antibody fragments forming a paratope, lectins, aptameres, peptides specific for predetermined MHC I or MHC II, MHC tetrameres, biotin and avidin.

15. Use of a combination according to one of the preceding claims for the analysis of biological samples, **characterized by** the subsequent contacting of the sample with a detection conjugate of a first analyte specificity, contacting the sample with the second reagent, and contacting the sample with a detection conjugate of a second analyte specificity.

16. Process for analysis of biological samples, **characterized by** the production of a combination according to one of the claims 1 to 14 in contact with the sample.

17. Process according to claim 16, comprising the steps
of contacting a biological sample with a detection conjugate having a binding portion (1) of a first specificity for a first analyte, a fluorochrome portion (5) connected to the binding portion (1), and a linker connected by its first end to the fluorochrome portion (5),
of detecting radiation emitted by the fluorochrome portion (5),
of deactivating the fluorochrome portion (5),
**characterized in that**
the linker is an oligonucleotide linker (4) which at least in a section is an oligonucleotide having a quencher section (4b) in its nucleic acid sequence, and
**in that** deactivating the fluorochrome portion (5) occurs by contacting the detection conjugate with a quencher (6) which is connected to that end of an oligonucleotide (8) hybridizeable to the quencher section (4b) which end upon hybridizing to the oligonucleotide linker (4) is located at its first end.

18. Process according to claim 16 or 17, **characterized by** the subsequent contacting of the sample with a detection conjugate of a first analyte specificity, contacting of the sample with a quencher specific for the first detection conjugate, and contacting of the sample with a detection conjugate of a second analyte specificity.

19. Process according to claim 18, **characterized in that** the fluorochrome portion (5) and the oligonucleotide linker (4) of detection conjugates used in subsequent contactings are identical to each other and each quencher (6) is the same having the same oligonucleotide (7).

20. Process according to claim 16 or 17, **characterized in that** deactivating the fluorochrome portion (5) occurs by contacting the detection conjugate with a nuclease and by subsequent removal of unbound components instead of the contacting with a quencher (6).

21. Process according to one of the claims 16 to 19, **characterized in that** the oligonucleotide linker (4) has a first oligonucleotide linker section (4a) which is bound to the binding portion (1) and in its nucleotide sequence has a fluorochrome section (4a), and **in that** the binding conjugate is produced by contacting the fluorochrome section (4a) with a fluorochrome portion (5) which is connected to a second oligonucleotide linker section (8) hybridizeable to the fluorochrome section.

22. Process according to claim 21, **characterized in that** the sample is contacted simultaneously with at least two detection conjugates, the fluorochrome sections (4a) of which are each unique.

23. Process according to one of the claims 16 to 22, **characterized in that** the sample is contacted simultaneously with at least two detection conjugates, the quencher sections (4b) of which are each unique.

24. Process according to one of the claims 16 to 23, **characterized in that** analytes are fixed on a carrier substrate.

25. Process according to claim 24, **characterized in that** the carrier substrate is part of a flow channel, in which the analytes are arranged for being flowed around by aqueous compositions.

## Revendications

1. Configuration de réactifs pour la détection d'analytes dans des spécimens, comprenant un premier réactif, présentant un conjugué de détection pourvu d'une portion de liaison (1) spécifique pour un analyte, une portion de fluorochrome (5) liée à la portion de liaison (1) et un liant oligonucléotide (4) liée en sa première extrémité à la portion de fluorochrome, liant étant au moins par sections un oligonucléotide, présentant une section de trempe (4b) dans sa séquence d'acide nucléique, et
un second réactif, présentant un quencher (6), qui est relié à l'extrémité d'un oligonucléotide (7) pouvant s'hybrider à la section de trempe (4b), oligonucléotide situé lors de l'hybridation avec le liant oligonucléotide (4) au niveau de sa première extrémité.

2. Configuration selon la revendication 1, **caractérisée en ce que** le liant oligonucléotide (4) est lié en sa seconde extrémité, opposée à la première extrémité, avec la portion de liant (1).

3. Configuration selon l'une des revendications précédentes, **caractérisée en ce que** le liant oligonucléotide (4) présente une première section de liant oligonucléotide (4a), section liée à la portion de liaison (1), où la première section de liant oligonucléotide (4a) présente une section de fluorochrome (4a) dans sa séquence d'acide nucléique, et **en ce que** la portion de fluorochrome (5) est liée à une seconde section de liant oligonucléotide (8) pouvant s'hybrider à la section de fluorochrome (4a).

4. Configuration selon la revendication 3, **caractérisée en ce que** la seconde section de liant oligonucléotide (8), liée à la portion de fluorochrome (5), présente la section de trempe (4b).

5. Configuration selon la revendication 4, **caractérisée en ce qu'**au moins deux conjugués de détection sont contenus, présentant chacun des sections de fluorochrome (4a) aux séquences nucléotides identiques ou similaires, avec lesquelles la même seconde section de liant oligonucléotide (8) peut s'hybrider dans des conditions physiologiques.

6. Configuration selon la revendication 4, **caractérisée en ce qu'**au moins deux conjugués de détection sont contenus, présentant chacun des sections de fluorochrome (4a) aux séquences nucléotides identiques ou similaires, avec lesquelles la même seconde section de liant oligonucléotide (8) peut s'hybrider dans des conditions physiologiques.

7. Configuration selon l'une des revendications précédentes, **caractérisée en ce qu'**au moins deux conjugués de détection sont contenus, présentant chacun des sections de trempe (4b) aux séquences nucléotides singulières.

8. Configuration selon l'une des revendications 1 à 6, **caractérisée en ce qu'**au moins deux conjugués de détection sont contenus, présentant chacun des sections de fluorochrome (4b) aux séquences nucléotides identiques ou similaires, avec lesquelles dans des conditions physiologiques le même oligonucléotide (7) liée au quencher (6) peut s'hybrider.

9. Configuration selon l'une des revendications précédentes, **caractérisée en ce que** le second réactif n'est pas un quencher (6) lié à l'oligonucléotide (7) et qu'éventuellement le liant oligonucléotide (4) ne présente aucune section de trempe (4b), et que le second réactif de la configuration est un nucléase.

10. Configuration selon la revendication 9, **caractérisée en ce que** le liant oligonucléotide (4) présente une séquence de reconnaissance pour un restrictase et que le nucléase est un restrictase spécifique pour la séquence de reconnaissance.

11. Configuration selon l'une des revendications précédentes, **caractérisée en ce qu'**indépendamment l'un de l'autre le liant oligonucléotide (4) et/ou l'oligonucléotide (7) lié au quencher (6) est un acide oligoribonucléique (ARN) monobrin, un acide oligodésoxyribonucléique (ADN) ou un acide oligopeptide nucléique (APN).

12. Configuration selon l'une des revendications 3 à 11, **caractérisée en ce qu'**indépendamment l'un de l'autre la première section de liant oligonucléotide (4a) du liant oligonucléotide (4) et/ou la seconde section de liant oligonucléotide (8) pouvant s'hybrider à celui-ci, section liée à la portion de fluorochrome (5), est un acide oligoribonucléique (ARN) monobrin, un acide oligodésoxyribonucléique (ADN) ou un acide oligopeptide nucléique (APN).

13. Configuration selon l'une des revendications précédentes, **caractérisée en ce que** deux ou plusieurs liants oligonucléotides (4) sont liés à la portion de liaison (1).

14. Configuration selon l'une des revendications précédentes, **caractérisée en ce que** la portion de liaison (1) est choisie parmi le groupe, consistant en anticorps, fragments d'anticorps constituant un paratope, lectines, aptamères, peptides, spécifiques pour des MHCI ou MHCII prédéterminés, MHC-tétramères, biotine et avidine.

15. Utilisation d'une configuration selon l'une des revendications précédentes pour l'analyse de spécimens biologiques, **caractérisée par** la mise en contact successive du spécimen avec un conjugué de détection d'une première spécificité d'analyte, mise en contact du spécimen avec le second réactif, et mise en contact du spécimen avec un conjugué de détection d'une seconde spécificité d'analyte.

16. Procédé d'analyse de spécimens biologiques, **caractérisé par** la fabrication d'une configuration selon l'une des revendications 1 à 14 au contact du spécimen.

17. Procédé selon la revendication 16, comprenant les phases de mise en contact d'un spécimen biologique avec un conjugué de détection, présentant une portion de liaison (1) d'une première spécificité pour un premier analyte, une portion de fluorochrome (5) liée à la portion de liaison (1) et un liant liée en sa première extrémité à la portion de fluorochrome (5),
détection d'un rayonnement émis par la portion de fluorochrome (5),
neutralisation de la portion de fluorochrome (5),
**caractérisée en ce**
**que** le liant est un liant oligonucléotide (4), liant étant au moins par sections un oligonucléotide, présentant une section de trempe (4b) dans sa séquence d'acide nucléique, et
**que** la neutralisation de la portion de fluorochrome (5) passe par la mise en contact du conjugué de détection avec un quencher (6), qui est relié à l'extrémité d'un oligonucléotide (8) pouvant s'hybrider à la section de trempe (4b), oligonucléotide situé lors de l'hybridation avec le liant oligonucléotide (4) au niveau de sa première extrémité.

18. Procédé selon la revendication 16 ou 17, **caractérisé par** la mise en contact successive du spécimen avec un conjugué de détection d'une première spécificité d'analyte, mise en contact du spécimen avec un quencher spécifique pour le premier conjugué de détection et mise en contact du spécimen avec un conjugué de détection d'une seconde spécificité d'analyte.

19. Procédé selon la revendication 18, **caractérisé en ce que** la portion de fluorochrome (5) et le liant oligonucléotide (4) sont respectivement identiques l'un à l'autre dans des conjugués de détection utilisés dans des mises en contact successives et que le quencher (6) est respectivement le même avec le même oligonucléotide (7).

20. Procédé selon la revendication 16 ou 17, **caractérisé en ce que** la neutralisation de la portion de fluorochrome (5) se produit à la place de la mise en contact avec un quencher (6) par la mise en contact du conjugué de détection avec un nucléase puis suppression des composantes non liées.

21. Procédé selon l'une quelconque des revendications 16 ou 19, **caractérisé en ce que** le liant oligonucléotide (4) présente une première section de liant oligonucléotide (4a), section liée à la portion de liaison (1) et présentant une section de fluorochrome (4a) dans sa séquence nucléotide et **en ce que** le conjugué de liaison est généré par la mise en contact de la section de fluorochrome (4a) avec une portion de fluorochrome (5), portion est liée à une seconde section de liant oligonucléotide (8) pouvant s'hybrider à la section de fluorochrome.

22. Procédé selon la revendication 21, **caractérisé en ce que** le spécimen est mis en contact simultanément avec au moins deux conjugués de détection dont les sections de fluorochrome (4a) sont chacune singulières.

23. Procédé selon l'une quelconque des revendications 16 à 22, **caractérisé en ce que** le spécimen est mis en contact simultanément avec au moins deux conjugués de détection dont les sections de trempe (4b) sont chacune singulières.

24. Procédé selon l'une quelconque des revendications 16 à 23, **caractérisé en ce que** des analytes sont fixés sur un substrat porteur.

25. Procédé selon la revendication 24, **caractérisé en ce que** le substrat porteur est la composante d'un canal d'écoulement, dans lequel les analytes peuvent être entourés de liquide par des compositions aqueuses.
